(19) Europäisches Patentamt
European Patent Office
Office européen des brevets

(11) Publication number: 0 135 191
B1

(12) # EUROPEAN PATENT SPECIFICATION

(45) Date of publication of patent specification: 26.10.88

(21) Application number: 84110916.8

(22) Date of filing: 12.09.84

(51) Int. Cl.⁴: **C 07 C 49/813**, C 07 C 49/84, C 07 C 79/36, C 07 C 69/757, A 01 N 35/06, A 01 N 37/42, C 07 C 143/78, C 07 C 147/06, C 07 C 147/10

(54) **Certain 2-(2-substituted benzoyl)-1,3-cyclohexanediones.**

(30) Priority: 17.08.84 US 640791
27.12.83 US 566077
16.09.83 US 532882

(43) Date of publication of application:
27.03.85 Bulletin 85/13

(45) Publication of the grant of the patent:
26.10.88 Bulletin 88/43

(84) Designated Contracting States:
AT BE CH DE FR GB IT LI NL SE

(56) References cited:
EP-A-0 007 243
EP-A-0 017 195
EP-A-0 090 262

CHEMICAL ABSTRACTS, vol. 85, 1976, p. 423, no. 5280f, Columbus, Ohio (US)
SYNTHESIS, December 1978, pages 925-926, Georg Thieme Publishers; A.A. AKHREM et al.: "A new simple synthesis of 2-acylcyclohexane-1,3-diones"

**The file contains technical information submitted after the application was filed and not included in this specification**

(73) Proprietor: STAUFFER CHEMICAL COMPANY
Westport Connecticut 06881 (US)

(72) Inventor: Michaely, William James
3161 Birmingham Drive
Richmond, Ca. 94806 (US)
Inventor: Kraatz, Gary Wayne
1423 Bing Drive
San José, CA 94806 (US)

(74) Representative: Kraus, Walter, Dr. et al
Patentanwälte Kraus, Weisert & Partner
Thomas-Wimmer-Ring 15
D-8000 München 22 (DE)

EP 0 135 191 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to 2-(2-substituted benzoyl)-1,3-cyclohexanediones, a method of controlling undesirable vegetation and a herbicidal composition comprising a 2-(2-substituted benzoyl)-1,3-cyclohexandione.

Compounds having the structural formula

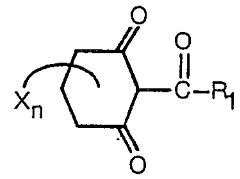

wherein X can be an alkyl, n can be 0, 1 or 2, and $R_1$ can be phenyl or substituted phenyl are described in Japanese patent application 84632—1974 as being intermediates for the preparation of herbicidal compounds of the formula

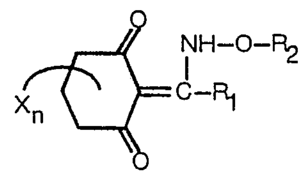

wherein $R_1$, X, and n are as defined above and $R_2$ is alkyl, alkenyl, or alkynyl. Specifically taught herbicidal compounds of this latter group are those where n is 2, X is 5,5-dimethyl, $R_2$ is allyl and $R_1$ is phenyl, 4-chlorophenyl or 4-methoxyphenyl.

The precursor intermediates for these three specifically taught compounds have no or almost no herbicidal activity.

In contrast, the compounds of this invention have exceptional herbicidal activity. Applicant's compounds must have a chlorine, bromine, iodine or alkoxy substitution in the 2-position of the phenyl moiety of their compounds to obtain the exceptional herbicidal activity. Chlorine is the preferred substituent. The exact reason why such a substitution imparts exceptional herbicidal activity to the compound is not fully understood.

EP—A3—90 262 which was published on October 5, 1983 is prior art in re novelty for the compounds of this invention. It is no prior art for the compounds in re inventive step which were described in U.S. patent application 532 882 of September 16, 1983, the first priority date of this application.

The compounds described and claimed in this application are not described in the above-mentioned European patent application.

This invention relates to certain novel 2-(2-substituted benzoyl)-cyclohexane-1,3-diones as herbicides. The compounds of this invention have the following structural formula

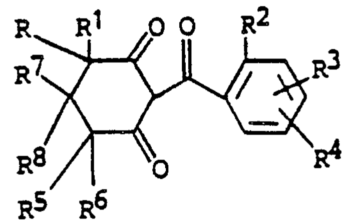

I

wherein

R is $C_1$—$C_6$ alkyl;

$R^1$ is hydrogen, $C_1$—$C_6$ alkyl, or

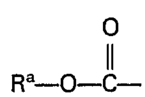

wherein $R^a$ is $C_1$—$C_4$ alkyl or R and $R^1$ together are alkylene having 3 to 6 carbon atoms;

$R^2$ is chlorine, bromine, iodine, or $C_1$—$C_4$ alkoxy;

$R^3$ and $R^4$ independently are

(1) hydrogen;

(2) halogen;

(3) $C_1$—$C_4$ alkyl;

(4) $C_1$—$C_4$ aliphatic alkoxy;

(5) nitro;
(6) $C_1$—$C_4$ haloalkyl; and
(7) $R^bSO_n$— wherein $R^b$ is $C_1$—$C_4$ alkyl; and n is the integer 0, 1 or 2;
$R^5$ is hydrogen or $C_1$—$C_6$ alkyl; and
$R^6$ is hydrogen or $C_1$—$C_6$ alkyl; and
$R^7$ is hydrogen or $C_1$—$C_6$ alkyl; and
$R^8$ is hydrogen or $C_1$—$C_6$ alkyl;
and their salts.

In the above formula R is preferably $C_1$—$C_4$ alkyl, more preferably methyl. $R^1$ is preferably $C_1$—$C_4$ alkyl, more preferably methyl, most preferably $R^1$ is hydrogen or methyl. $R^2$ is preferably methoxy, most preferably $R^2$ is chlorine, bromine or methoxy. $R^3$ and $R^4$ are preferably hydrogen, fluorine, chlorine or bromine, methyl, methoxy, trifluoromethyl. If $R^3$ is $R^bSO_n$—, $R^b$ is preferably methyl, and n is preferably 2.

$R^5$, $R^6$, $R^7$ and $R^8$ are independently the same or different and are selected from the group consisting of hydrogen, $C_1$—$C_6$ alkyl, preferably $C_1$—$C_4$ alkyl, more preferably methyl, most preferably hydrogen or methyl, and their salts.

More preferably, $R^3$ is chlorine, hydrogen, methyl, alkylthio or methoxy. Preferably $R^4$ is hydrogen, chlorine, nitro, $CF_3$, or $R^bSO_n$ wherein $R^b$ is $C_1$—$C_4$ alkyl, preferably methyl and n is the integer 0, 1 or 2, preferably 2.

The compounds of this invention can have the following four structural formulae because of tautomerism:

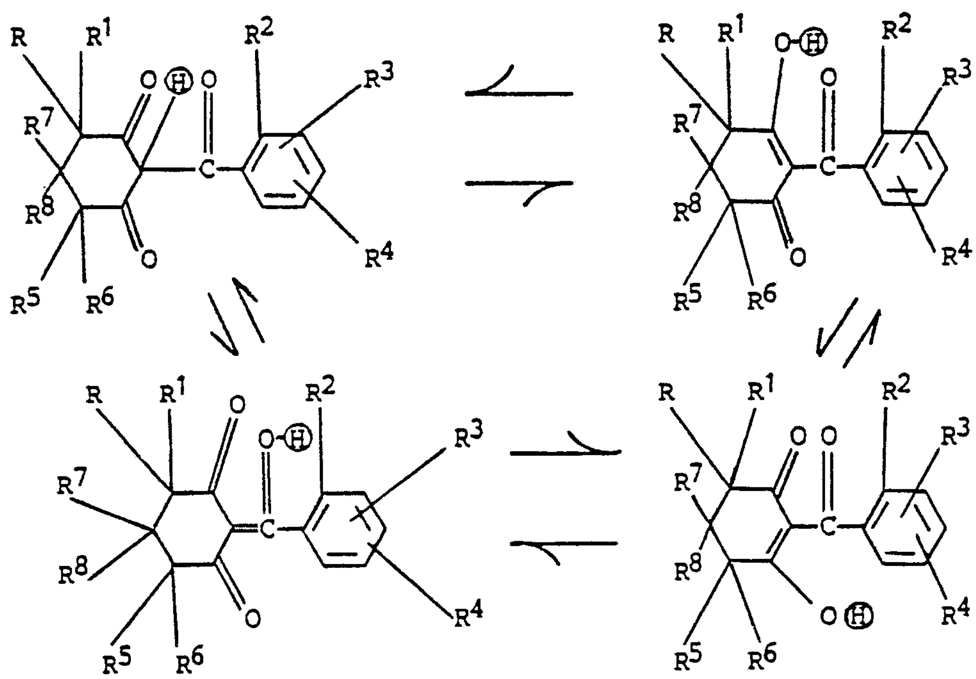

wherein R, $R^1$, $R^2$, $R^3$, $R^4$, $R^5$, $R^6$, $R^7$ and $R^8$ are as defined above.

The circled proton on each of the four tautomers is reasonably labile. These protons are acidic and can be removed by any base to give a salt having an anion of the following four resonance forms:

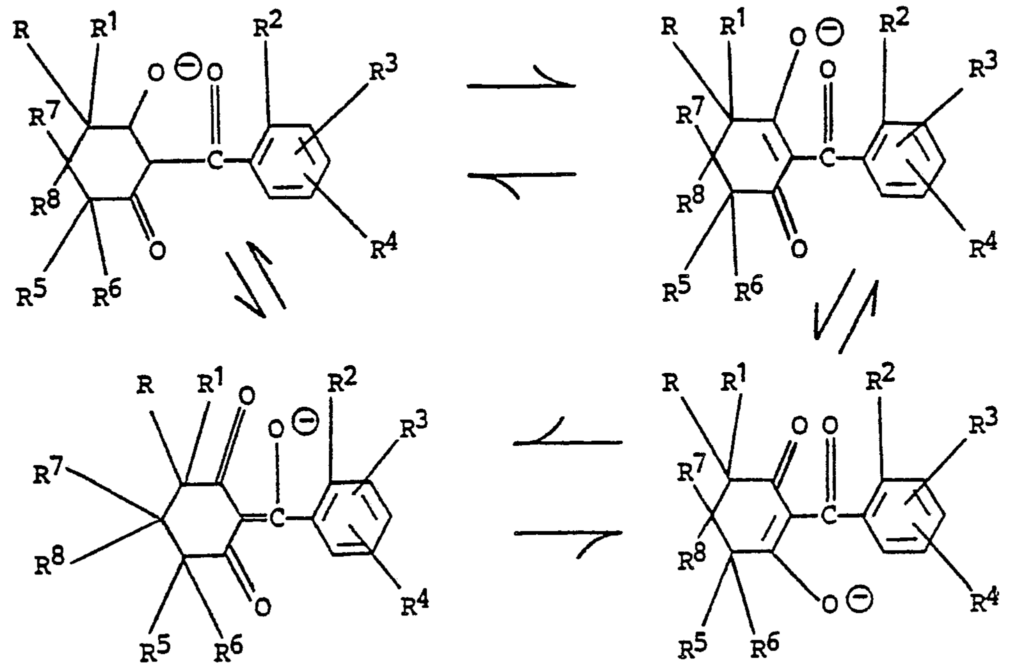

wherein R, $R^1$, $R^2$, $R^3$, $R^4$, $R^5$, $R^6$, $R^7$ and $R^8$ are as defined above.

Examples of cations of these bases are inorganic cations such as alkali metals e.g. lithium, sodium, and potassium, alkaline earth metals, e.g. barium, magnesium, calcium and strontium, or organic cations such as substituted ammonium, sulfonium or phosphonium wherein the substituent is an aliphatic or aromatic group.

The term "aliphatic group" is used herein in a broad sense to cover a large class of organic groups characterized by being derived from (1) an acylic (open-chain structure) of the paraffin, olefin and acetylene hydrocarbon series and their derivatives or (2) alicyclic compounds. The aliphatic group can have from 1 to 10 carbon atoms.

The term "aromatic group" is used herein in a broad sense to distinguish from the aliphatic group and includes a group derived from (1) compounds having 6 to 20 carbon atoms and characterized by the presence of at least one benzene ring, including monocyclic, bicyclic and polycyclic hydrocarbons and their derivatives and (2) heterocyclic compounds having 5 to 19 carbon atoms which are similar in structure and are characterized by having an unsaturated ring structure containing at least one atom other than carbon, such as nitrogen, sulfur and oxygen and derivatives of these heterocyclic compounds.

In the above description of the compounds of this invention alkyl and alkoxy include both straight and branched configurations; for example, methyl, ethyl, n-propyl, isopropyl, n-butyl, sec-butyl, isobutyl, and tert-butyl.

The compounds of this invention and their salts are active herbicides of a general type. That is, they are herbicidally effective against a wide range of plant species. The method of controlling undesirable vegetation of the present invention comprises applying an herbicidally effective amount of the above-described compounds to the area where control is desired.

The compounds of the present invention can be prepared by the following general method.

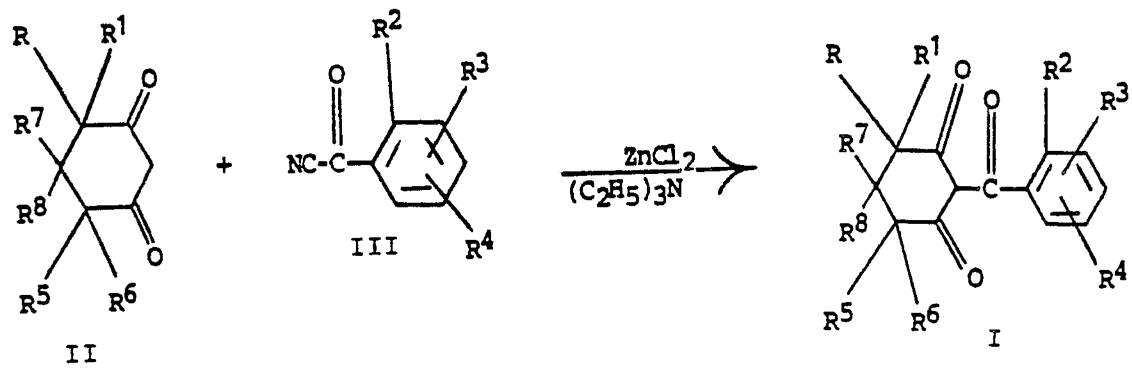

II III I

Generally, mole amounts of the dione and substituted benzoyl cyanide are used, along with a slight mole excess of zinc chloride. The two reactants and the zinc chloride are combined in a solvent such as

methylene chloride. A slight mole excess of triethylamine is slowly added to the reaction mixture with cooling. The mixture is stirred at room temperature for 5 hours.

The reaction product is worked up by conventional techniques.

The above-described substituted benzoyl cyanide can be prepared according to the teaching of T. S. Oakwood and C. A. Weisgerber, *Organic Synthesis Collected*, Vol. III, pp. 122 (1955).

The following example teaches the synthesis of a representative compound of this invention.

Example I

4,4-Dimethyl-2-(2,4-dichlorobenzyl)-cyclohexane-1,3-dione

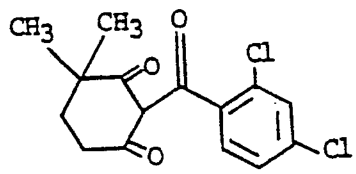

4,4-Dimethyl-1,3-cyclohexanedione [14.0 grams (g), 0.1 mole], 20.0 g (0.1 mole) 2,4-dichlorobenzoyl cyanide and 13.6 g (0.11 mole) anhydrous, powdered zinc chloride were combined in 100 milliliters (ml) methylene chloride. Triethylamine (10.1 g, 0.12 mole) was slowly added with cooling. The reaction mixture was stirred at room temperature for 5 hours and then poured into 2N hydrochloric acid. The aqueous phase was discarded and the organic phase was washed with 150 ml 5% $Na_2CO_3$ four times. The aqueous washings were combined and acidified with HCl, extracted with methylene chloride, dried and concentrated to yield 25.3 g of crude product. The crude product was chromatographed (2% $AcOH/CH_2Cl_2$) in 5 g aliquots then reduced on rotavap under a separator pressure at 50°C for 30 minutes to remove AcOH. This yielded an oil (40% yield). The structure was confirmed by instrumental analysis.

The following is a table of certain selected compounds that are preparable according to the procedure described hereto. Compound numbers are assigned to each compound and are used throughout the remainder of the application.

TABLE I

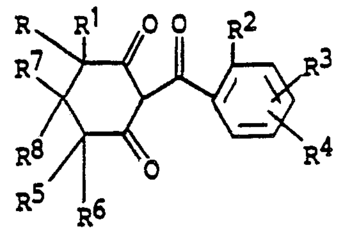

| Compound Number | R | $R^1$ | $R^2$ | $R^3$ | $R^4$ | $R^5$ | $R^6$ | $R^7$ | $R^8$ |
|---|---|---|---|---|---|---|---|---|---|
| 1 | $C_2H_5-O-\overset{O}{\overset{\|}{C}}-$ | H | Cl | H | 4-Cl | H | H | H | H |
| 2 | $C_2H_5-O-\overset{O}{\overset{\|}{C}}-$ | $CH_3$ | Cl | H | 4-Cl | H | H | H | H |
| 3 | triethanolammonium salt of Compound No. 2 | | | | | | | | |
| 4 | triethanolammonium salt of Compound No. 7 | | | | | | | | |
| 5 | $C_2H_5-O-\overset{O}{\overset{\|}{C}}-$ | $CH_3$ | Cl | 3-Cl | 4-Cl | H | H | H | H |
| 6 | triethanolammonium salt of Compound No. 5 | | | | | | | | |
| 7 | $CH_3$ | $CH_3$ | Cl | H | 4-Cl | H | H | H | H |
| 8 | $CH_3$ | $CH_3$ | Cl | H | 4-Cl | $CH_3$ | H | H | H |
| 9 | $CH_3$ | n-$C_3H_7$ | Cl | H | 4-Cl | H | H | H | H |
| 10 | $CH_3$ | n-$C_3H_7$ | Cl | H | 4-Cl | $CH_3$ | H | H | H |
| 11 | $CH_3$ | n-$C_4H_9$ | Cl | H | 4-Cl | H | H | H | H |
| 12 | $CH_3$ | n-$C_4H_9$ | Cl | H | 4-Cl | $CH_3$ | H | H | H |
| 13 | $CH_3$ | H | Cl | H | 4-Cl | H | H | H | H |
| 14 | $CH_3$ | H | Cl | H | 4-Cl | $CH_3$ | H | H | H |
| 15 | $C_2H_5$ | H | Cl | H | 4-Cl | H | H | H | H |
| 16 | $C_2H_5$ | H | Cl | H | 4-Cl | $CH_3$ | H | H | H |
| 17 | n-$C_4H_9$ | H | Cl | H | 4-Cl | H | H | H | H |
| 18 | n-$C_4H_9$ | H | Cl | H | 4-Cl | $CH_3$ | H | H | H |
| 19 | i-$C_3H_7$ | H | Cl | H | 4-Cl | H | H | H | H |
| 20 | i-$C_3H_7$ | H | Cl | H | 4-Cl | $CH_3$ | H | H | H |
| 21 | $-C_5H_{10}-$ | | Cl | H | 4-Cl | H | H | H | H |
| 22 | $-C_5H_{10}-$ | | Cl | H | 4-Cl | $CH_3$ | H | H | H |
| 23 | $CH_3$ | $CH_3$ | Cl | 3-Cl | 4-Cl | H | H | H | H |

TABLE I
(continued)

| Compound Number | R | $R^1$ | $R^2$ | $R^3$ | $R^4$ | $R^5$ | $R^6$ | $R^7$ | $R^8$ |
|---|---|---|---|---|---|---|---|---|---|
| 24 | $CH_3$ | $CH_3$ | Cl | 3-Cl | 4-Cl | $CH_3$ | H | H | H |
| 25 | $CH_3$ | n-$C_3H_7$ | Cl | 3-Cl | 4-Cl | H | H | H | H |
| 26 | $CH_3$ | n-$C_3H_7$ | Cl | 3-Cl | 4-Cl | $CH_3$ | H | H | H |
| 27 | $CH_3$ | n-$C_4H_9$ | Cl | 3-Cl | 4-Cl | H | H | H | H |
| 28 | $CH_3$ | n-$C_4H_9$ | Cl | 3-Cl | 4-Cl | $CH_3$ | H | H | H |
| 29 | $CH_3$ | H | Cl | 3-Cl | 4-Cl | H | H | H | H |
| 30 | $CH_3$ | H | Cl | 3-Cl | 4-Cl | $CH_3$ | H | H | H |
| 31 | $C_2H_5$ | H | Cl | 3-Cl | 4-Cl | H | H | H | H |
| 32 | $C_2H_5$ | H | Cl | 3-Cl | 4-Cl | $CH_3$ | H | H | H |
| 33 | n-$C_4H_9$ | H | Cl | 3-Cl | 4-Cl | H | H | H | H |
| 34 | n-$C_4H_9$ | H | Cl | 3-Cl | 4-Cl | $CH_3$ | H | H | H |
| 35 | i-$C_3H_7$ | H | Cl | 3-Cl | 4-Cl | H | H | H | H |
| 36 | i-$C_3H_7$ | H | Cl | 3-Cl | 4-Cl | $CH_3$ | H | H | H |
| 37 | $-C_5H_{10}-$ | | Cl | 3-Cl | 4-Cl | H | H | H | H |
| 38 | $-C_5H_{10}-$ | | Cl | 3-Cl | 4-Cl | $CH_3$ | H | H | H |
| 39 | $CH_3$ | $CH_3$ | Cl | H | 4-$CH_3SO_2$ | H | H | H | H |
| 40 | $CH_3$ | $CH_3$ | Cl | H | 4-$CH_3SO_2$ | $CH_3$ | H | H | H |
| 41 | $CH_3$ | n-$C_3H_7$ | Cl | H | 4-$CH_3SO_2$ | H | H | H | H |
| 42 | $CH_3$ | n-$C_3H_7$ | Cl | H | 4-$CH_3SO_2$ | $CH_3$ | H | H | H |
| 43 | $CH_3$ | n-$C_4H_9$ | Cl | H | 4-$CH_3SO_2$ | H | H | H | H |
| 44 | $CH_3$ | n-$C_4H_9$ | Cl | H | 4-$CH_3SO_2$ | $CH_3$ | H | H | H |
| 45 | $CH_3$ | H | Cl | H | 4-$CH_3SO_2$ | H | H | H | H |
| 46 | $CH_3$ | H | Cl | H | 4-$CH_3SO_2$ | $CH_3$ | H | H | H |
| 47 | $C_2H_5$ | H | Cl | H | 4-$CH_3SO_2$ | H | H | H | H |
| 48 | $C_2H_5$ | H | Cl | H | 4-$CH_3SO_2$ | $CH_3$ | H | H | H |
| 49 | n-$C_4H_9$ | H | Cl | H | 4-$CH_3SO_2$ | H | H | H | H |
| 50 | n-$C_4H_9$ | H | Cl | H | 4-$CH_3SO_2$ | $CH_3$ | H | H | H |
| 51 | i-$C_3H_7$ | H | Cl | H | 4-$CH_3SO_2$ | H | H | H | H |
| 52 | i-$C_3H_7$ | H | Cl | H | 4-$CH_3SO_2$ | $CH_3$ | H | H | H |
| 53 | $-C_5H_{10}-$ | | Cl | H | 4-$CH_3SO_2$ | H | H | H | H |
| 54 | $-C_5H_{10}-$ | | Cl | H | 4-$CH_3SO_2$ | $CH_3$ | H | H | H |
| 55 | $CH_3$ | $CH_3$ | Cl | 3-Cl | 4-$CH_3SO_2$ | H | H | H | H |

TABLE I
(continued)

| Compound Number | R | $R^1$ | $R^2$ | $R^3$ | $R^4$ | $R^5$ | $R^6$ | $R^7$ | $R^8$ |
|---|---|---|---|---|---|---|---|---|---|
| 56 | $CH_3$ | $CH_3$ | Cl | 3-Cl | $4\text{-}CH_3SO_2$ | $CH_3$ | H | H | H |
| 57 | $CH_3$ | $n\text{-}C_3H_7$ | Cl | 3-Cl | $4\text{-}CH_3SO_2$ | H | H | H | H |
| 58 | $CH_3$ | $n\text{-}C_3H_7$ | Cl | 3-Cl | $4\text{-}CH_3SO_2$ | $CH_3$ | H | H | H |
| 59 | $CH_3$ | $n\text{-}C_4H_9$ | Cl | 3-Cl | $4\text{-}CH_3SO_2$ | H | H | H | H |
| 60 | $CH_3$ | $n\text{-}C_4H_9$ | Cl | 3-Cl | $4\text{-}CH_3SO_2$ | $CH_3$ | H | H | H |
| 61 | $CH_3$ | H | Cl | 3-Cl | $4\text{-}CH_3SO_2$ | H | H | H | H |
| 62 | $CH_3$ | H | Cl | 3-Cl | $4\text{-}CH_3SO_2$ | $CH_3$ | H | H | H |
| 63 | $C_2H_5$ | H | Cl | 3-Cl | $4\text{-}CH_3SO_2$ | H | H | H | H |
| 64 | $C_2H_5$ | H | Cl | 3-Cl | $4\text{-}CH_3SO_2$ | $CH_3$ | H | H | H |
| 65 | $n\text{-}C_4H_9$ | H | Cl | 3-Cl | $4\text{-}CH_3SO_2$ | H | H | H | H |
| 66 | $n\text{-}C_4H_9$ | H | Cl | 3-Cl | $4\text{-}CH_3SO_2$ | $CH_3$ | H | H | H |
| 67 | $i\text{-}C_3H_7$ | H | Cl | 3-Cl | $4\text{-}CH_3SO_2$ | H | H | H | H |
| 68 | $i\text{-}C_3H_7$ | H | Cl | 3-Cl | $4\text{-}CH_3SO_2$ | $CH_3$ | H | H | H |
| 69 | $—C_5H_{10}—$ | | Cl | 3-Cl | $4\text{-}CH_3SO_2$ | H | H | H | H |
| 70 | $—C_5H_{10}—$ | | Cl | 3-Cl | $4\text{-}CH_3SO_2$ | $CH_3$ | H | H | H |
| 71 | $CH_3$ | $CH_3$ | Cl | 3-Cl | $4\text{-}C_2H_5SO_2$ | H | H | H | H |
| 72 | $CH_3$ | $CH_3$ | Cl | 3-Cl | $4\text{-}C_2H_5SO_2$ | $CH_3$ | H | H | H |
| 73 | $CH_3$ | $n\text{-}C_3H_7$ | Cl | 3-Cl | $4\text{-}C_2H_5SO_2$ | H | H | H | H |
| 74 | $CH_3$ | $n\text{-}C_3H_7$ | Cl | 3-Cl | $4\text{-}C_2H_5SO_2$ | $CH_3$ | H | H | H |
| 75 | $CH_3$ | $n\text{-}C_4H_9$ | Cl | 3-Cl | $4\text{-}C_2H_5SO_2$ | H | H | H | H |
| 76 | $CH_3$ | $n\text{-}C_4H_9$ | Cl | 3-Cl | $4\text{-}C_2H_5SO_2$ | $CH_3$ | H | H | H |
| 77 | $CH_3$ | H | Cl | 3-Cl | $4\text{-}C_2H_5SO_2$ | H | H | H | H |
| 78 | $CH_3$ | H | Cl | 3-Cl | $4\text{-}C_2H_5SO_2$ | $CH_3$ | H | H | H |
| 79 | $C_2H_5$ | H | Cl | 3-Cl | $4\text{-}C_2H_5SO_2$ | H | H | H | H |
| 80 | $C_2H_5$ | H | Cl | 3-Cl | $4\text{-}C_2H_5SO_2$ | $CH_3$ | H | H | H |
| 81 | $n\text{-}C_4H_9$ | H | Cl | 3-Cl | $4\text{-}C_2H_5SO_2$ | H | H | H | H |
| 82 | $n\text{-}C_4H_9$ | H | Cl | 3-Cl | $4\text{-}C_2H_5SO_2$ | $CH_3$ | H | H | H |
| 83 | $i\text{-}C_3H_7$ | H | Cl | 3-Cl | $4\text{-}C_2H_5SO_2$ | H | H | H | H |
| 84 | $i\text{-}C_3H_7$ | H | Cl | 3-Cl | $4\text{-}C_2H_5SO_2$ | $CH_3$ | H | H | H |
| 85 | $—C_5H_{10}—$ | | Cl | 3-Cl | $4\text{-}C_2H_5SO_2$ | H | H | H | H |
| 86 | $—C_5H_{10}—$ | | Cl | 3-Cl | $4\text{-}C_2H_5SO_2$ | $CH_3$ | H | H | H |
| 87 | $CH_3$ | $CH_3$ | Cl | H | $4\text{-}C_2H_5SO_2$ | H | H | H | H |

TABLE I
(continued)

| Compound Number | R | $R^1$ | $R^2$ | $R^3$ | $R^4$ | $R^5$ | $R^6$ | $R^7$ | $R^8$ |
|---|---|---|---|---|---|---|---|---|---|
| 88 | $CH_3$ | $CH_3$ | Cl | H | 4-$C_2H_5SO_2$ | $CH_3$ | H | H | H |
| 89 | $CH_3$ | n-$C_3H_7$ | Cl | H | 4-$C_2H_5SO_2$ | H | H | H | H |
| 90 | $CH_3$ | n-$C_3H_7$ | Cl | H | 4-$C_2H_5SO_2$ | $CH_3$ | H | H | H |
| 91 | $CH_3$ | n-$C_4H_9$ | Cl | H | 4-$C_2H_5SO_2$ | H | H | H | H |
| 92 | $CH_3$ | n-$C_4H_9$ | Cl | H | 4-$C_2H_5SO_2$ | $CH_3$ | H | H | H |
| 93 | $CH_3$ | H | Cl | H | 4-$C_2H_5SO_2$ | H | H | H | H |
| 94 | $CH_3$ | H | Cl | H | 4-$C_2H_5SO_2$ | $CH_3$ | H | H | H |
| 95 | $C_2H_5$ | H | Cl | H | 4-$C_2H_5SO_2$ | H | H | H | H |
| 96 | $C_2H_5$ | H | Cl | H | 4-$C_2H_5SO_2$ | $CH_3$ | H | H | H |
| 97 | n-$C_4H_9$ | H | Cl | H | 4-$C_2H_5SO_2$ | H | H | H | H |
| 98 | n-$C_4H_9$ | H | Cl | H | 4-$C_2H_5SO_2$ | $CH_3$ | H | H | H |
| 99 | i-$C_3H_7$ | H | Cl | H | 4-$C_2H_5SO_2$ | H | H | H | H |
| 100 | i-$C_3H_7$ | H | Cl | H | 4-$C_2H_5SO_2$ | $CH_3$ | H | H | H |
| 101 | —$C_5H_{10}$— | | Cl | H | 4-$C_2H_5SO_2$ | H | H | H | H |
| 102 | —$C_5H_{10}$— | | Cl | H | 4-$C_2H_5SO_2$ | $CH_3$ | H | H | H |
| 103 | $CH_3$ | $CH_3$ | Cl | 3-$OCH_3$ | 4-$C_2H_5SO_2$ | H | H | H | H |
| 104 | $CH_3$ | $CH_3$ | Cl | 3-$OCH_3$ | 4-$C_2H_5SO_2$ | $CH_3$ | H | H | H |
| 105 | $CH_3$ | n-$C_3H_7$ | Cl | 3-$OCH_3$ | 4-$C_2H_5SO_2$ | H | H | H | H |
| 106 | $CH_3$ | n-$C_3H_7$ | Cl | 3-$OCH_3$ | 4-$C_2H_5SO_2$ | $CH_3$ | H | H | H |
| 107 | $CH_3$ | n-$C_4H_9$ | Cl | 3-$OCH_3$ | 4-$C_2H_5SO_2$ | H | H | H | H |
| 108 | $CH_3$ | n-$C_4H_9$ | Cl | 3-$OCH_3$ | 4-$C_2H_5SO_2$ | $CH_3$ | H | H | H |
| 109 | $CH_3$ | H | Cl | 3-$OCH_3$ | 4-$C_2H_5SO_2$ | H | H | H | H |
| 110 | $CH_3$ | H | Cl | 3-$OCH_3$ | 4-$C_2H_5SO_2$ | $CH_3$ | H | H | H |
| 111 | $C_2H_5$ | H | Cl | 3-$OCH_3$ | 4-$C_2H_5SO_2$ | H | H | H | H |
| 112 | $C_2H_5$ | H | Cl | 3-$OCH_3$ | 4-$C_2H_5SO_2$ | $CH_3$ | H | H | H |
| 113 | n-$C_4H_9$ | H | Cl | 3-$OCH_3$ | 4-$C_2H_5SO_2$ | H | H | H | H |
| 114 | n-$C_4H_9$ | H | Cl | 3-$OCH_3$ | 4-$C_2H_5SO_2$ | $CH_3$ | H | H | H |
| 115 | i-$C_3H_7$ | H | Cl | 3-$OCH_3$ | 4-$C_2H_5SO_2$ | H | H | H | H |
| 116 | i-$C_3H_7$ | H | Cl | 3-$OCH_3$ | 4-$C_2H_5SO_2$ | $CH_3$ | H | H | H |
| 117 | —$C_5H_{10}$— | | Cl | 3-$OCH_3$ | 4-$C_2H_5SO_2$ | H | H | H | H |
| 118 | —$C_5H_{10}$— | | Cl | 3-$OCH_3$ | 4-$C_2H_5SO_2$ | $CH_3$ | H | H | H |
| 119 | $CH_3$ | $CH_3$ | Cl | 3-$CH_3$ | 4-$C_2H_5SO_2$ | H | H | H | H |

TABLE I
(continued)

| Compound Number | R | $R^1$ | $R^2$ | $R^3$ | $R^4$ | $R^5$ | $R^6$ | $R^7$ | $R^8$ |
|---|---|---|---|---|---|---|---|---|---|
| 120 | $CH_3$ | $CH_3$ | Cl | 3-$CH_3$ | 4-$C_2H_5SO_2$ | $CH_3$ | H | H | H |
| 121 | $CH_3$ | n-$C_3H_7$ | Cl | 3-$CH_3$ | 4-$C_2H_5SO_2$ | H | H | H | H |
| 122 | $CH_3$ | n-$C_3H_7$ | Cl | 3-$CH_3$ | 4-$C_2H_5SO_2$ | $CH_3$ | H | H | H |
| 123 | $CH_3$ | n-$C_4H_9$ | Cl | 3-$CH_3$ | 4-$C_2H_5SO_2$ | H | H | H | H |
| 124 | $CH_3$ | n-$C_4H_9$ | Cl | 3-$CH_3$ | 4-$C_2H_5SO_2$ | $CH_3$ | H | H | H |
| 125 | $CH_3$ | H | Cl | 3-$CH_3$ | 4-$C_2H_5SO_2$ | H | H | H | H |
| 126 | $CH_3$ | H | Cl | 3-$CH_3$ | 4-$C_2H_5SO_2$ | $CH_3$ | H | H | H |
| 127 | $C_2H_5$ | H | Cl | 3-$CH_3$ | 4-$C_2H_5SO_2$ | H | H | H | H |
| 128 | $C_2H_5$ | H | Cl | 3-$CH_3$ | 4-$C_2H_5SO_2$ | $CH_3$ | H | H | H |
| 129 | n-$C_4H_9$ | H | Cl | 3-$CH_3$ | 4-$C_2H_5SO_2$ | H | H | H | H |
| 130 | n-$C_4H_9$ | H | Cl | 3-$CH_3$ | 4-$C_2H_5SO_2$ | $CH_3$ | H | H | H |
| 131 | i-$C_3H_7$ | H | Cl | 3-$CH_3$ | 4-$C_2H_5SO_2$ | H | H | H | H |
| 132 | i-$C_3H_7$ | H | Cl | 3-$CH_3$ | 4-$C_2H_5SO_2$ | $CH_3$ | H | H | H |
| 133 | $—C_5H_{10}—$ | | Cl | 3-$CH_3$ | 4-$C_2H_5SO_2$ | H | H | H | H |
| 134 | $—C_5H_{10}—$ | | Cl | 3-$CH_3$ | 4-$C_2H_5SO_2$ | $CH_3$ | H | H | H |
| 135 | $CH_3$ | $CH_3$ | Cl | 3-$OCH_3$ | 4-$CH_3SO_2$- | H | H | H | H |
| 136 | $CH_3$ | $CH_3$ | Cl | 3-$OCH_3$ | 4-$CH_3SO_2$- | $CH_3$ | H | H | H |
| 137 | $CH_3$ | n-$C_3H_7$ | Cl | 3-$OCH_3$ | 4-$CH_3SO_2$ | H | H | H | H |
| 138 | $CH_3$ | n-$C_3H_7$ | Cl | 3-$OCH_3$ | 4-$CH_3SO_2$- | $CH_3$ | H | H | H |
| 139 | $CH_3$ | n-$C_4H_9$ | Cl | 3-$OCH_3$ | 4-$CH_3SO_2$- | H | H | H | H |
| 140 | $CH_3$ | n-$C_4H_9$ | Cl | 3-$OCH_3$ | 4-$CH_3SO_2$- | $CH_3$ | H | H | H |
| 141 | $CH_3$ | H | Cl | 3-$OCH_3$ | 4-$CH_3SO_2$- | H | H | H | H |
| 142 | $CH_3$ | H | Cl | 3-$OCH_3$ | 4-$CH_3SO_2$- | $CH_3$ | H | H | H |
| 143 | $C_2H_5$ | H | Cl | 3-$OCH_3$ | 4-$CH_3SO_2$- | H | H | H | H |
| 144 | $C_2H_5$ | H | Cl | 3-$OCH_3$ | 4-$CH_3SO_2$- | $CH_3$ | H | H | H |
| 145 | n-$C_4H_9$ | H | Cl | 3-$OCH_3$ | 4-$CH_3SO_2$- | H | H | H | H |
| 146 | n-$C_4H_9$ | H | Cl | 3-$OCH_3$ | 4-$CH_3SO_2$- | $CH_3$ | H | H | H |
| 147 | i-$C_3H_7$ | H | Cl | 3-$OCH_3$ | 4-$CH_3SO_2$- | H | H | H | H |
| 148 | i-$C_3H_7$ | H | Cl | 3-$OCH_3$ | 4-$CH_3SO_2$- | $CH_3$ | H | H | H |
| 149 | $—C_5H_{10}—$ | | Cl | 3-$OCH_3$ | 4-$CH_3SO_2$- | H | H | H | H |
| 150 | $—C_5H_{10}—$ | | Cl | 3-$OCH_3$ | 4-$CH_3SO_2$- | $CH_3$ | H | H | H |
| 151 | $CH_3$ | $CH_3$ | Cl | 3-$CH_3$ | 4-$CH_3SO_2$- | H | H | H | H |

TABLE I

(continued)

| Compound Number | R | $R^1$ | $R^2$ | $R^3$ | $R^4$ | $R^5$ | $R^6$ | $R^7$ | $R^8$ |
|---|---|---|---|---|---|---|---|---|---|
| 152 | $CH_3$ | $CH_3$ | Cl | 3-$CH_3$ | 4-$CH_3SO_2$- | $CH_3$ | H | H | H |
| 153 | $CH_3$ | n-$C_3H_7$ | Cl | 3-$CH_3$ | 4-$CH_3SO_2$- | H | H | H | H |
| 154 | $CH_3$ | n-$C_3H_7$ | Cl | 3-$CH_3$ | 4-$CH_3SO_2$- | $CH_3$ | H | H | H |
| 155 | $CH_3$ | n-$C_4H_9$ | Cl | 3-$CH_3$ | 4-$CH_3SO_2$- | H | H | H | H |
| 156 | $CH_3$ | n-$C_4H_9$ | Cl | 3-$CH_3$ | 4-$CH_3SO_2$- | $CH_3$ | H | H | H |
| 157 | $CH_3$ | H | Cl | 3-$CH_3$ | 4-$CH_3SO_2$- | H | H | H | H |
| 158 | $CH_3$ | H | Cl | 3-$CH_3$ | 4-$CH_3SO_2$- | $CH_3$ | H | H | H |
| 159 | $C_2H_5$ | H | Cl | 3-$CH_3$ | 4-$CH_3SO_2$- | H | H | H | H |
| 160 | $C_2H_5$ | H | Cl | 3-$CH_3$ | 4-$CH_3SO_2$- | $CH_3$ | H | H | H |
| 161 | n-$C_4H_9$ | H | Cl | 3-$CH_3$ | 4-$CH_3SO_2$- | H | H | H | H |
| 162 | n-$C_4H_9$ | H | Cl | 3-$CH_3$ | 4-$CH_3SO_2$- | $CH_3$ | H | H | H |
| 163 | i-$C_3H_7$ | H | Cl | 3-$CH_3$ | 4-$CH_3SO_2$- | H | H | H | H |
| 164 | i-$C_3H_7$ | H | Cl | 3-$CH_3$ | 4-$CH_3SO_2$- | $CH_3$ | H | H | H |
| 165 | $-C_5H_{10}-$ | | Cl | 3-$CH_3$ | 4-$CH_3SO_2$ | | H | H | H |
| 166 | $-C_5H_{10}-$ | | Cl | 3-$CH_3$ | 4-$CH_3SO_2$- | $CH_3$ | H | H | H |
| 167 | $CH_3$ | $CH_3$ | Cl | 3-$CH_3$ | 4-Cl | H | H | H | H |
| 168 | $CH_3$ | $CH_3$ | Cl | 3-$CH_3$ | 4-Cl | $CH_3$ | H | H | H |
| 169 | $CH_3$ | n-$C_3H_7$ | Cl | 3-$CH_3$ | 4-Cl | H | H | H | H |
| 170 | $CH_3$ | n-$C_3H_7$ | Cl | 3-$CH_3$ | 4-Cl | $CH_3$ | H | H | H |
| 171 | $CH_3$ | n-$C_4H_9$ | Cl | 3-$CH_3$ | 4-Cl | H | H | H | H |
| 172 | $CH_3$ | n-$C_4H_9$ | Cl | 3-$CH_3$ | 4-Cl | $CH_3$ | H | H | H |
| 173 | $CH_3$ | H | Cl | 3-$CH_3$ | 4-Cl | H | H | H | H |
| 174 | $CH_3$ | H | Cl | 3-$CH_3$ | 4-Cl | $CH_3$ | H | H | H |
| 175 | $C_2H_5$ | H | Cl | 3-$CH_3$ | 4-Cl | H | H | H | H |
| 176 | $C_2H_5$ | H | Cl | 3-$CH_3$ | 4-Cl | $CH_3$ | H | H | H |
| 177 | n-$C_4H_9$ | H | Cl | 3-$CH_3$ | 4-Cl | H | H | H | H |
| 178 | n-$C_4H_9$ | H | Cl | 3-$CH_3$ | 4-Cl | $CH_3$ | H | H | H |
| 179 | i-$C_3H_7$ | H | Cl | 3-$CH_3$ | 4-Cl | H | H | H | H |
| 180 | i-$C_3H_7$ | H | Cl | 3-$CH_3$ | 4-Cl | $CH_3$ | H | H | H |
| 181 | $-C_5H_{10}-$ | | Cl | 3-$CH_3$ | 4-Cl | H | H | H | H |
| 182 | $-C_5H_{10}-$ | | Cl | 3-$CH_3$ | 4-Cl | $CH_3$ | H | H | H |
| 183 | $CH_3$ | $CH_3$ | Cl | 3-$OCH_3$ | 4-Cl | H | H | H | H |

TABLE I

(continued)

| Compound Number | R | $R^1$ | $R^2$ | $R^3$ | $R^4$ | $R^5$ | $R^6$ | $R^7$ | $R^8$ |
|---|---|---|---|---|---|---|---|---|---|
| 184 | $CH_3$ | $CH_3$ | Cl | $3-OCH_3$ | 4-Cl | $CH_3$ | H | H | H |
| 185 | $CH_3$ | $n-C_3H_7$ | Cl | $3-OCH_3$ | 4-Cl | H | H | H | H |
| 186 | $CH_3$ | $n-C_3H_7$ | Cl | $3-OCH_3$ | 4-Cl | $CH_3$ | H | H | H |
| 187 | $CH_3$ | $n-C_4H_9$ | Cl | $3-OCH_3$ | 4-Cl | H | H | H | H |
| 188 | $CH_3$ | $n-C_4H_9$ | Cl | $3-OCH_3$ | 4-Cl | $CH_3$ | H | H | H |
| 189 | $CH_3$ | H | Cl | $3-OCH_3$ | 4-Cl | H | H | H | H |
| 190 | $CH_3$ | H | Cl | $3-OCH_3$ | 4-Cl | $CH_3$ | H | H | H |
| 191 | $C_2H_5$ | H | Cl | $3-OCH_3$ | 4-Cl | H | H | H | H |
| 192 | $C_2H_5$ | H | Cl | $3-OCH_3$ | 4-Cl | $CH_3$ | H | H | H |
| 193 | $n-C_4H_9$ | H | Cl | $3-OCH_3$ | 4-Cl | H | H | H | H |
| 194 | $n-C_4H_9$ | H | Cl | $3-OCH_3$ | 4-Cl | $CH_3$ | H | H | H |
| 195 | $i-C_3H_7$ | H | Cl | $3-OCH_3$ | 4-Cl | H | H | H | H |
| 196 | $i-C_3H_7$ | H | Cl | $3-OCH_3$ | 4-Cl | $CH_3$ | H | H | H |
| 197 | $—C_5H_{10}—$ | | Cl | $3-OCH_3$ | 4-Cl | H | H | H | H |
| 198 | $—C_5H_{10}—$ | | Cl | $3-OCH_3$ | 4-Cl | $CH_3$ | H | H | H |
| 199 | $CH_3$ | $C_2H_5$ | Cl | H | 4-Cl | H | H | H | H |
| 200 | $CH_3$ | $CH_3$ | Cl | H | H | H | H | H | H |
| 201 | $CH_3$ | $CH_3$ | Cl | $3-OC_2H_5$ | Br | H | H | H | H |
| 202 | $CH_3$ | $CH_3$ | Cl | H | 4-Br | H | H | H | H |
| 203 | $CH_3$ | $C_2H_5$ | Cl | 3-Cl | 4-Cl | H | H | H | H |
| 204 | $n-C_3H_7$ | H | Cl | H | 4-Cl | H | H | H | H |
| 205 | $C_2H_5O\overset{O}{C}-$ | $n-C_3H_7$ | Cl | H | 4-Cl | H | H | H | H |
| 206 | $CH_3$ | $CH_3$ | Cl | H | $4-i-C_3H_7SO_2$ | H | H | H | H |
| 207 | $CH_3$ | $CH_3$ | Cl | $4-iC_3H_7O$ | 4-Br | H | H | H | H |
| 208 | $CH_3$ | $CH_3$ | Cl | H | 6-F | H | H | H | H |
| 209 | $i-C_3H_7$ | H | Cl | 3-Cl | 4-Cl | H | H | H | H |
| 210 | $CH_3$ | $CH_3$ | Cl | $3-OC_2H_5$ | 4-Br | H | H | H | H |
| 211 | $i-C_3H_7OC(O)$ | H | Cl | H | 4-Cl | H | H | H | H |
| 212 | $C_2H_5OC(O)-$ | $n-C_3H_7$ | Cl | H | 4-Cl | H | H | H | H |
| 213 | $—C_5H_{10}—$ | | Cl | H | 4-Cl | H | H | H | H |
| 214 | $CH_3$ | $CH_3$ | Cl | H | 4-Cl | H | H | $CH_3$ | H |

TABLE I
(continued)

| Compound Number | R | $R^1$ | $R^2$ | $R^3$ | $R^4$ | $R^5$ | $R^6$ | $R^7$ | $R^8$ |
|---|---|---|---|---|---|---|---|---|---|
| 215 | $CH_3$ | $CH_3$ | Cl | H | 4-n-$C_3H_7SO_2$- | H | H | H | H |
| 216 | $CH_3$ | $CH_3$ | Cl | 3-Cl | 4-n-$C_3H_7SO_2$- | H | H | H | H |
| 217 | $CH_3$ | $CH_3$ | $CH_3O$ | 3-$CH_3O$ | H | H | H | H | H |
| 218 | $CH_3$ | $CH_3$ | $CH_3O$ | H | 4-Cl | H | H | H | H |
| 219 | $CH_3$ | $CH_3$ | Cl | H | 4-Br | $CH_3$ | H | H | H |
| 220 | $CH_3$ | $CH_3$ | Br | H | H | H | H | H | H |
| 221 | $CH_3$ | $CH_3$ | I | H | H | H | H | H | H |
| 222 | $CH_3$ | $CH_3$ | F | H | H | H | H | H | H |
| 223 | $CH_3$ | $CH_3$ | $CH_3O$ | H | H | H | H | H | H |
| 224 | $CH_3$ | $CH_3$ | Cl | 3-allyloxy | 4-Br | H | H | H | H |
| 225 | $CH_3$ | $CH_3$ | Cl | H | 4-$CH_3SO_2$ | H | H | $CH_3$ | H |
| 226 | $CH_3$ | $CH_3$ | Cl | 3-$CH_3O$ | 4-Br | $CH_3$ | H | H | H |
| 227 | $CH_3$ | $CH_3$ | Br | H | 3-CN | H | H | H | H |
| 228 | $CH_3$ | $CH_3$ | Cl | H | 4N($CH_3$)$SO_2CF_3$ | H | H | H | H |
| 229 | $CH_3$ | $CH_3$ | Cl | 3-$NO_2$ | H | H | H | H | H |
| 230 | $CH_3$ | $CH_3$ | $C_2H_5O$ | H | 4-Cl | H | H | H | H |
| 231 | $CH_3$ | $CH_3$ | Cl | H | * | H | H | H | H |
| 232 | $CH_3$ | $CH_3$ | Cl | 3-$C_2H_5O$ | 4-$CH_3SO_2$ | H | H | H | H |
| 233 | $CH_3$ | $CH_3$ | Cl | 3-$CH_3O$ | 4-$C_2H_5SO_2$ | H | H | H | H |
| 234 | $CH_3$ | $CH_3$ | Cl | H | 4-n-$C_4H_9SO_2$ | H | H | H | H |
| 235 | $CH_3$ | $CH_3$ | Cl | 3-Cl | 4-n-$C_4H_9SO_2$ | $CH_3$ | H | H | H |
| 236 | $CH_3$ | $CH_3$ | Cl | 3-Cl | 4-$C_2H_5SO_2$ | $CH_3$ | H | H | H |
| 237 | $CH_3$ | $CH_3$ | Cl | H | 4-F | H | H | H | H |
| 238 | $CH_3$ | H | Cl | 3-$CH_3O$ | 4-Br | H | H | H | H |
| 239 | $CH_3$ | $CH_3$ | Cl | 3-$CH_3O$ | H | H | H | H | H |
| 240 | $CH_3$ | $CH_3$ | Cl | EtS | $PrSO_2$ | H | H | H | H |
| 241 | $CH_3$ | $CH_3$ | Cl | EtS | EtS | H | H | H | H |
| 242 | $CH_3$ | $CH_3$ | Cl | EtS | $EtSO_2$ | H | H | H | H |
| 243 | $CH_3$ | $CH_3$ | Cl | EtS | $MeSO_2$ | H | H | H | H |

* = 4-butylsulfinyl

Herbicidal Screening Tests

As previously mentioned, the herein described compounds produced in the above-described manner are phytotoxic compounds which are useful and valuable in controlling various plant species. Selected compounds of this invention were tested as herbicides in the following manner.

Pre-Emergence Herbicide Test

On the day preceding treatment, seeds of eight different weed species are planted in loamy sand soil in individual rows using one species per row across the width of a flat. The seeds used are green foxtail (FT) (*Setaria viridis*), watergrass (WG) (*Echinochloa crusgalli*), wild oat (WO) (*Avena fatua*), annual morningglory (AMG) (*Ipomoea lacunosa*), velvetleaf (VL) (*Abutilon theophrasti*), Indian mustard (MD) (*Brassica juncea*), redroot pigweed (PW) (*Amaranthus retroflexus*) or curly dock (CD) (*Rumex crispus*), and yellow nutsedge (YNG) (*Cyperus esculentus*). Ample seeds are planted to give about 20 to 40 seedlings per row, after emergence, depending upon the size of the plants.

Using an analytical balance, 600 milligrams (mg) of the compound to be tested are weighed out on a piece of glassine weighing paper. The paper and compound are placed in a 60 milliliter (ml) wide-mouth clear bottle and dissolved in 45 ml of acetone or substituted solvent. Eighteen ml of this solution are transferred to a 60 ml wide-mouth clear bottle and diluted with 22 ml of a water and acetone mixture (19:1) containing enough polyoxyethylene sorbitan monolaurate emulsifier to give a final solution of 0.5% (v/v). The solution is then sprayed on a seeded flat on a linear spray table calibrated to deliver 80 gallons per acre (748 L/ha). The application rate is 4 lb/acre (4.48 Kg/ha).

After treatment, the flats are placed in the greenhouse at a temperature of 70 to 80°F and watered by sprinkling. Two weeks after treatment, the degree of injury or control is determined by comparison with untreated check plants of the same age. The injury rating from 0 to 100% is recorded for each species as percent control with 0% representing no injury and 100% representing complete control.

The results of the tests are shown in the following Table II.

TABLE II

Pre-Emergence Herbicidal Activity
Application Rate — 4.48 kg/ha

| Cmpd. No. | FT | WG | WO | AMG | VL | MD | CD | PW | YNG |
|---|---|---|---|---|---|---|---|---|---|
| 1 | 0 | 85 | 10 | 75 | 100 | 80 | 80 | | 75 |
| 2 | 90 | 90 | 80 | 10 | 90 | 90 | — | | 80 |
| 3 | | | | | | | | | |
| 4 | 100 | 100 | 80 | 60 | 100 | 80 | 90 | | 100 |
| 5 | 80 | 100 | 20 | 40 | 100 | 100 | 80 | | 100 |
| 6 | 40 | 100 | 0 | 40 | 100 | 100 | 80 | | 100 |
| 7 | 100 | 100 | 100 | 80 | 100 | 100 | 90 | | 100 |
| 8 | 100 | 100 | 60 | 45 | 60 | 60 | 80 | | 100 |
| 9 | 60 | 100 | 90 | 20 | 60 | 60 | 60 | | 100 |
| 13 | 100 | 100 | 40 | 5 | 80 | 60 | 80 | | 100 |
| 14 | 80 | 90 | 60 | 95 | 90 | 90 | 100 | | 80 |
| 21 | 25 | 95 | 10 | 20 | 20 | 20 | 60 | | 40 |
| 23 | 100 | 100 | 90 | 90 | 100 | 100 | 100 | | 100 |
| 35 | 100 | 60 | 50 | 80 | 90 | 90 | 40 | | 60 |
| 39 | 60 | 60 | 80 | 80 | 90 | 90 | 60 | | 65 |
| 40 | 100 | 100 | 100 | 100 | 100 | 100 | 100 | | 97 |
| 45 | 100 | 100 | 85 | 100 | 100 | 100 | 90 | | 95 |
| 55 | 100 | 80 | 85 | 100 | 100 | 90 | 100 | | 70 |
| 71 | 90 | 65 | 90 | 85 | 70 | 60 | 100 | | 40 |
| 88 | 40 | 70 | 80 | 90 | 90 | 100 | 95 | | 40 |
| 167 | 40 | 70 | 40 | 40 | 40 | 60 | 70 | | 50 |
| 183 | 64 | 70 | 80 | 40 | 90 | 80 | 70 | | 80 |
| 199 | 20 | 95 | 60 | 5 | 100 | 100 | 90 | | 100 |
| 200 | 100 | 100 | 80 | 20 | 90 | 40 | 80 | | 100 |
| 201 | 100 | 100 | 70 | 20 | 100 | 100 | 100 | | 100 |
| 202 | 100 | 100 | 80 | 60 | 100 | 100 | 80 | | 100 |
| 203 | 100 | 100 | 0 | 10 | 100 | 100 | 100 | | 100 |
| 204 | 90 | 70 | 10 | 0 | 0 | 0 | 80 | | 10 |
| 205 | 0 | 10 | 0 | 0 | 10 | 10 | 0 | | 0 |
| 206 | 100 | 100 | 90 | 80 | 100 | 100 | 90 | | 100 |
| 207 | 100 | 100 | 90 | 40 | 90 | 90 | 90 | | 90 |
| 208 | 100 | 100 | 85 | 0 | 95 | 85 | 95 | | 90 |
| 209 | 90 | 90 | 50 | 20 | 100 | 100 | 100 | | 80 |
| 211 | 90 | 80 | 25 | 30 | 30 | 45 | 50 | | 90 |
| 212 | 0 | 10 | 0 | 0 | 10 | 10 | 0 | | 0 |
| 213 | 0 | 20 | 0 | 0 | 10 | 20 | 10 | | 0 |
| 214 | 100 | 100 | 90 | 45 | 100 | 100 | 65 | | 100 |
| 215 | 80 | 90 | 95 | 95 | 100 | 100 | 100 | | 5 |
| 216 | 100 | 95 | 95 | 95 | 40 | 100 | 100 | | 30 |
| 217 | 30 | 65 | 0 | 30 | 45 | 40 | 65 | | 50 |
| 218 | 70 | 75 | 0 | 65 | 100 | 50 | 50 | | 70 |
| 219 | 100 | 100 | 100 | 98 | 100 | 100 | 100 | | 85 |
| 220 | 100 | 100 | 85 | 15 | 100 | 100 | 85 | | 95 |
| 221 | 100 | 100 | 65 | 0 | 100 | 100 | 50 | | 50 |
| 222 | 80 | 80 | 55 | 0 | 98 | 55 | 80 | | 65 |
| 223 | 95 | 95 | 10 | 0 | 65 | 0 | 25 | | 60 |
| 224 | 100 | 100 | 100 | 100 | 100 | 100 | 100 | | 50 |
| 225 | 100 | 100 | 75 | 100 | 100 | 100 | 100 | | 50 |

TABLE II
(continued)

| Cmpd. No. | FT | WG | WO | AMG | VL | MD | CD | PW | YNG |
|---|---|---|---|---|---|---|---|---|---|
| 226 | 100 | 100 | 60 | 15 | 100 | 100 | 100 | | 80 |
| 227 | 100 | 100 | 55 | 15 | 100 | 100 | 90 | | 95 |
| 228 | 100 | 100 | 60 | 20 | 95 | 100 | 90 | | 10 |
| 229 | 5 | 10 | 0 | 0 | 40 | 20 | 0 | | 0 |
| 230 | 10 | 40 | 10 | 10 | 95 | 40 | 100 | | 80 |
| 231 | 100 | 100 | 75 | 80 | 100 | 100 | 100 | | 75 |
| 232 | 100 | 100 | 90 | 100 | 100 | 100 | 85 | | - |
| 233 | 100 | 100 | 80 | 100 | 100 | 100 | 100 | | - |
| 234 | 95 | 100 | 50 | 95 | 100 | 100 | 85 | | - |
| 235 | 100 | 100 | 70 | 100 | 90 | 100 | 100 | | - |
| 236 | 100 | 100 | 75 | 100 | 100 | 100 | 85 | | - |
| 237 | 100 | 100 | 55 | 40 | 100 | 100 | 90 | | 7 |
| 238 | 100 | 100 | 65 | 95 | 100 | 100 | 100 | | 95 |
| 239 | 100 | 100 | 30 | 15 | 100 | 100 | 95 | | 90 |

— = Species did not germinate for some reason.
A blank indicates that the weed was not tested.

Post-Emergence Herbicide Test

This test is conducted in an identical manner to the testing procedure for the pre-emergence herbicide test, except the seeds of the eight different weed species are planted 1012 days before treatment. Also, watering of the treated flats is confined to the soil surface and not to the foliage of the sprouted plants.

The results of the post-emergence herbicide test are reported in Table III.

TABLE III

Post-Emergence Herbicidal Activity
Application Rate — 4.48 kg/ha

| Cmpd. No. | FT | WG | WO | AMG | VL | MD | CD | PW | YNG |
|---|---|---|---|---|---|---|---|---|---|
| 1 | 85 | 95 | 20 | 95 | 100 | 100 | 95 | | 95 |
| 2 | 90 | 100 | 65 | 100 | 100 | 100 | 100 | | 100 |
| 3 | | | | | | | | | |
| 4 | 60 | 60 | 90 | 40 | 60 | 60 | 90 | | 70 |
| 5 | 40 | 60 | 10 | 20 | 40 | 40 | 90 | | 80 |
| 6 | 40 | 50 | 10 | 60 | 20 | 20 | 60 | | 50 |
| 7 | 80 | 80 | 80 | 60 | 60 | 90 | 60 | | 60 |
| 8 | 100 | 80 | 60 | 30 | 80 | 80 | 80 | | 80 |
| 9 | 70 | 70 | 60 | 40 | 60 | 60 | 80 | | 70 |
| 13 | 40 | 40 | 60 | 30 | 60 | 60 | 90 | | 60 |
| 14 | 100 | 100 | 90 | 10 | 20 | 20 | 90 | | 100 |
| 21 | 0 | 20 | 0 | 0 | 10 | 20 | 10 | | 0 |
| 23 | 100 | 100 | 95 | 70 | 60 | 60 | 80 | | 100 |
| 35 | 90 | 90 | 50 | 20 | 100 | 100 | 100 | | 80 |
| 39 | 100 | 100 | 95 | 100 | 100 | 100 | 90 | | 100 |
| 40 | 100 | 70 | 90 | 100 | – | 90 | 98 | | 70 |
| 45 | 100 | 85 | 100 | 85 | – | 100 | 100 | | 65 |
| 55 | 100 | 100 | 95 | 100 | 100 | 100 | 100 | | 95 |
| 71 | 100 | 100 | 85 | 100 | 100 | 100 | 100 | | 90 |
| 88 | 100 | 100 | 80 | 100 | 100 | 100 | 100 | | 90 |
| 167 | 100 | 100 | 75 | 80 | 100 | 100 | 100 | | 90 |
| 183 | 100 | 100 | 65 | 30 | 100 | 100 | 100 | | 90 |
| 199 | 100 | 80 | 60 | 20 | 90 | 90 | 80 | | 45 |
| 200 | 90 | 60 | 60 | 60 | 60 | 60 | 40 | | 70 |
| 201 | 90 | 70 | 70 | 40 | 70 | 70 | 60 | | 60 |
| 202 | 100 | 90 | 70 | 50 | 100 | 100 | 80 | | 80 |
| 203 | 100 | 100 | 10 | 20 | 20 | 20 | 80 | | 60 |
| 204 | 50 | 30 | 10 | 0 | 0 | 0 | 60 | | 0 |
| 205 | 20 | 40 | 10 | 60 | 90 | 40 | 60 | | 40 |
| 206 | 80 | 80 | 80 | 80 | 100 | 100 | 80 | | 50 |
| 207 | 60 | 50 | 50 | 20 | 60 | 60 | 80 | | 60 |
| 208 | 100 | 80 | 80 | 75 | – | 100 | 100 | | 95 |
| 209 | 100 | 60 | 50 | 80 | 90 | 90 | 40 | | 60 |
| 211 | 15 | 100 | 0 | 50 | 100 | 80 | 70 | | 40 |
| 212 | 20 | 40 | 10 | 60 | 90 | 90 | 60 | | 40 |
| 213 | 25 | 45 | 10 | 20 | 20 | 20 | 60 | | 45 |
| 214 | 90 | 80 | 80 | 80 | 100 | 100 | 100 | | 70 |
| 215 | 100 | 100 | 90 | 100 | 100 | 100 | 100 | | 90 |
| 216 | 100 | 100 | 85 | 100 | 100 | 100 | 100 | | 95 |
| 217 | 65 | 100 | 0 | 0 | 100 | 20 | 20 | | 90 |
| 218 | 100 | 100 | 0 | 20 | 100 | 100 | 98 | | 95 |
| 219 | 100 | 100 | 90 | 85 | 100 | 100 | 100 | | 95 |
| 220 | 100 | 65 | 80 | 50 | 100 | 100 | 60 | | 60 |
| 221 | 60 | 70 | 70 | 40 | 80 | 70 | 30 | | 50 |
| 222 | 0 | 60 | 40 | 60 | 100 | 90 | 20 | | 30 |
| 223 | 100 | 75 | 80 | 40 | 100 | 50 | 20 | | 30 |
| 224 | 90 | 100 | 100 | 100 | 100 | 100 | 100 | | 40 |
| 225 | 60 | 70 | 70 | 70 | 90 | 65 | 40 | | 60 |

TABLE III

(continued)

| Cmpd. No. | FT | WG | WO | AMG | VL | MD | CD | PW | YNG |
|---|---|---|---|---|---|---|---|---|---|
| 226 | 100 | 100 | 85 | 100 | 100 | 100 | 100 | | 60 |
| 227 | 95 | 85 | 90 | 100 | 100 | 100 | 90 | | 45 |
| 228 | 85 | 100 | 0 | 10 | 15 | 95 | 40 | | 50 |
| 229 | 85 | 70 | 65 | 0 | 0 | 0 | 0 | | 35 |
| 230 | 20 | 40 | 10 | 15 | 70 | 40 | 35 | | 40 |
| 231 | 100 | 95 | 100 | 95 | 100 | 95 | 100 | | 40 |
| 232 | 65 | 75 | 75 | 80 | 90 | 85 | 80 | | 60 |
| 233 | 80 | 80 | 70 | 95 | 80 | 85 | 80 | | 50 |
| 234 | 100 | 80 | 25 | 80 | 75 | 80 | 85 | | 35 |
| 235 | 100 | 100 | 40 | 95 | 95 | 100 | 95 | | 50 |
| 236 | 75 | 70 | 50 | 90 | 90 | 100 | 90 | | 50 |
| 237 | 100 | 80 | 80 | 60 | 85 | 60 | 0 | | - |
| 238 | 98 | 90 | 60 | 35 | - | 100 | 60 | | 90 |
| 239 | 70 | 65 | 20 | 15 | 95 | 40 | 70 | | 45 |

Pre-Emergence Multi-Weed Herbicide Test

Several compounds were evaluated at an application rate of 2 lb/acre (2.24 kg/ha) for pre-emergence activity against a larger number of weed species:

The process was generally similar to the pre-emergence herbicide test described above except that only 300 milligrams of test compound were weighed out and the application rate was 40 gallons per acre.

Redroot pigweed (PW) and curly dock (CD) were eliminated in this test and the following weed species were added:

| | | | |
|---|---|---|---|
| *Grasses:* | downybrome | *Bromus tectorum* | (DB) |
| | annual ryegrass | *Lolium multiflorum* | (ARG) |
| | rox-orange sorghum | *Sorghum bicolor* | (SHC) |
| | hemp sesbania | *Sesbania exaltata* | (SESB) |
| | nightshade | *Solanum sp.* | (SP) |
| | cocklebur | *Xattiium sp.* | (CB) |

The results of the test are shown in Table IV.

TABLE IV

Pre-Emergence Multi-Weed Herbicide Test

| Cmpd. No. | DB | FT | ARG | WG | SHC | WO | BSG | AMG | SESB | VL | SP | MD | YNS | CB |
|---|---|---|---|---|---|---|---|---|---|---|---|---|---|---|
| 24 | 80 | 100 | 100 | 100 | 100 | 80 | 95 | 70 | 60 | 100 | 40 | 85 | 100 | 10 |
| 210 | 100 | 100 | 100 | 100 | 100 | 100 | 100 | 20 | 10 | 100 | 20 | 95 | 100 | 20 |

The compounds of the present invention are useful as herbicides, especially as pre-emergence herbicides, and can be applied in a variety of ways at various concentrations. In practice, the compounds herein defined are formulated into herbicidal compositions, by admixture, in herbicidally effective amounts, with the adjuvants and carriers normally employed for facilitating the dispersion of active ingredients for agricultural applications, recognizing the fact that the formulation and mode of application of a toxicant may affect the activity of the materials in a given application. Thus, these active herbicidal compounds may be formulated as granules of relatively large particle size, as wettable powders, as emulsifiable concentrates, as powdery dusts, as solutions or as any of several other known types of

formulations, depending upon the desired mode of application. Preferred formulations for pre-emergence herbicidal applications are wettable powders, emulsifiable concentrates and granules. These formulations may contain at little as about 0.5% to as much as about 95% or more by weight of active ingredient. A herbicidally effective amount depends upon the nature of the seeds or plants to be controlled and the rate of application varies from about 0.05 to approximately 25 pounds per acre, preferably from about 0.1 to about 10 pounds per acre.

Wettable powders are in the form of finely divided particles which disperse readily in water or other dispersants. The wettable powder is ultimately applied to the soil either as a dry dust or as a dispersion in water or other liquid. Typical carriers for wettable powders include fuller's earth, kaolin clays, silicas and other readily wet organic or inorganic diluents. Wettable powders normally are prepared to contain about 5% to about 95% of the active ingredient and usually also contain a small amount of wetting, dispersing, or emulsifying agent to facilitate wetting and dispersion.

Emulsifiable concentrates are homogeneous liquid compositions which are dispersible in water or other dispersant, and may consist entirely of the active compound with a liquid or solid emulsifying agent, or may also contain a liquid carrier, such as xylene, heavy aromatic naphthal, isophorone and other nonvolatile organic solvents. For herbicidal application, these concentrates are dispersed in water or other liquid carrier and normally applied as a spray to the area to be treated. The percentage by weight of the essential active ingredient may vary according to the manner in which the composition is to be applied, but in general comprises about 0.5% to 95% of active ingredient by weight of the herbicidal composition.

Granular formulations wherein the toxicant is carried on relatively coarse particles, are usually applied without dilution to the area in which suppression of vegetation is desired. Typical carriers for granular formulations include sand, fuller's earth, bentonite clays, vermiculite, perlite and other organic or inorganic materials which absorb or which may be coated with the toxicant. Granular formulations normally are prepared to contain about 5% to about 25% of active ingredients which may include surface-active agents such heavy aromatic naphthas, kerosene or other petroleum fractions, or vegetable oils; and/or stickers such as destrins, glue or synthetic resins.

Typical wetting, dispersing or emulsifying agents used in agricultural formulations include, for example, the alkyl and alkylaryl sulfonates and sulfates and their sodium salts; polyhydric alcohols; and other types of surface-active agents, many of which are available in commerce. The surface-active agent, when used, normally comprises from 0.1% to 15% by weight of the herbicidal composition.

Dusts, which are free-flowing admixtures of the active ingredient with finely divided solids such as talc, clays, flours and other organic and inorganic solids which act as dispersants and carriers for the toxicant, are useful formulations for soil-incorporating application.

Pastes, which are homogeneous suspensions of a finely divided solid toxicant in a liquid carrier such as water or oil, are employed for specific purposes. These formulations normally contain about 5% to about 95% of active ingredient by weight, and may also contain small amounts of a wetting, dispersing or emulsifying agent to facilitate dispersion. For application, the pastes are normally diluted and applied as a spray to the area to be affected.

Other useful formulations for herbicidal applications include simple solutions of the active ingredient in a dispersant in which it is completely soluble at the desired concentration, such as acetone, alkylated naphthalenes, xylene and other organic solvents. Pressurized sprays, typically aerosols, wherein the active ingredient is dispersed in finely-divided form as a result of vaporization of a low boiling dispersant solvent carrier, such as the Freons, may also be used.

The phytotoxic compositions of this invention are applied to the plants in the conventional manner. Thus, the dust and liquid compositions can be applied to the plant by the use of power-dusters, boom and hand sprayers and spray dusters. The compositions can also be applied from airplanes as a dust or a spray because they are effective in very low dosages. In order to modify or control growth of germinating seeds or emerging seedlings, as a typical example, the dust and liquid compositions are applied to the soil according to conventional methods and are distributed in the soil to a depth of at least ½ inch below the soil surface. It is not necessary that the phytotoxic compositions be admixed with the soil particles since these compositions can also be applied merely by spraying or sprinkling the surface of the soil. The phytotoxic compositions of this invention can also be applied by addition to irrigation water supplied to the field to be treated. This method of application permits the penetration of the compositions into the soil as the water is absorbed therein. Dust compositions, granular compositions or liquid formulations applied to the surface of the soil can be distributed below the surface of the soil by conventional means such as discing, dragging or mixing operations.

The phytotoxic compositions of this invention can also contain other additaments, for example, fertilizers and other herbicides, pesticides and the like, used as adjuvant or in combination with any of the above-described adjuvants. Other phytotoxic compounds useful in combination with the above-described compounds include, for example, anilides such as 2-benzothiazole-2-yloxy-N-methyl acetanilide, 2-chloro-2',6'-dimethyl-N-(n-propylethyl)acetanilide, 2-chloro-2',6'-diethyl-N-(butoxymethyl)acetanilide; 2,4-dichlorophenoxyacetic acids, 2,4,5-trichlorophenoxyacetic acid, 2-methyl-4-chlorophenoxyacetic acid and the salts, esters and amides thereof; triazine derivatives, such as 2,4-bis(3-methoxypropylamino)-6-methylthio-s-triazine, 2-chloro-4-ethylamino-6-isopropylamino-s-triazine, and 2-ethylamino-4-isopropyl-amino-6-methyl-mercapto-s-triazine; urea derivatives, such as 3-(3,5-dichlorophenyl)-1,1-dimethylurea and 3-(p-chlorophenyl)-1,1-dimethylurea; and acetamides such as N,N-diallyl-α-chloroacetamide, and the like; benzoic acids such as 3-amino-2,5-dichlorobenzoic acid; thiocarbamates such as S-(1,1-dimethylbenzyl)-piperidene-1-carbothiaote, 3-(4-chlorophenyl)-methyl diethylcarbothioate, ethyl-1-hexahydro-1,4-azepine-1-carbothioate, S-ethyl-hexahydro-1H-azepine-1-carbothioate, S-propyl N,N-dipropylthiocarbamate, S-ethyl N,N-dipropylthiocarbamate, S-ethyl cyclohexylethylthiocarbamate and the like; anilines such as 4-(methylsulfonyl)-2,6-dinitro-N,N-substituted aniline, 4-trifluoromethyl-2,6-dinitro-N,N-di-n-propyl aniline, 4-trifluoromethyl-2,6-dinitro-N-ethyl-N-butyl aniline, 2-[4-(2,4-dichlorophenoxy)phenoxy]propanoic acid, 2-[1-(ethoxyimino)butyl]-5-[2-ethylthio)propyl]-3-hydroxy-2-cyclohexene-1-one, (±)-butyl-2[4-[(5-trifluoromethyl)-2-pyridinyl)oxy]phenoxy]propanate, sodium 5-[2-chloro-4-(trifluoromethyl)phenoxy]-2-nitrobenzoate, 3-isopropyl-1H-2,1,3-benzothiadiazine-4(3H)-one-2,2-dioxide, and 4-amino-6-tert-butyl-3(methylthio)-as-triazin-5(4H)-one or 4-amino-6-(1,1-dimethylethyl)-3-(methylthio)-1,2,4-triazin-5(4H)-one and S-(O,O-diisopropyl)-benzene sulfonamide. Fertilizers useful in combination with the active ingredients include, for example, ammonium nitrate, urea and superphosphate. Other useful additaments include materials in which plant organisms take root and grow such as compost, manure, humus, sand, and the like.

**Claims for the Contracting States: BE CH DE FR GB IT LI NL SE**

1. A compound having the structural formula

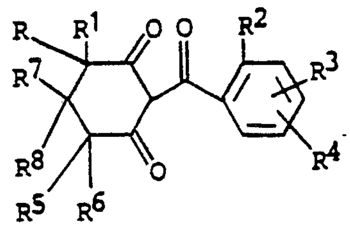

I

wherein
R is $C_1$—$C_6$ alkyl;
$R^1$ is hydrogen, $C_1$—$C_6$ alkyl, or

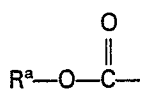

wherein $R^a$ is $C_1$—$C_4$ alkyl or R and $R^1$ together are alkylene having 3 to 6 carbon atoms;
$R^2$ is chlorine, bromine, iodine, or $C_1$—$C_4$ alkoxy;
$R^3$ and $R^4$ independently are
(1) hydrogen;
(2) halogen;
(3) $C_1$—$C_4$ alkyl;
(4) $C_1$—$C_4$ aliphatic alkoxy;
(5) nitro;
(6) $C_1$—$C_4$ haloalkyl; and
(7) $R^bSO_n$— wherein $R^b$ is $C_1$—$C_4$ alkyl; and
n is the integer 0, 1 or 2;
$R^5$ is hydrogen or $C_1$—$C_6$ alkyl; and
$R^6$ is hydrogen or $C_1$—$C_6$ alkyl; and
$R^7$ is hydrogen or $C_1$—$C_6$ alkyl; and
$R^8$ is hydrogen or $C_1$—$C_6$ alkyl;
and their salts.

2. The compounds of Claim 1 wherein:
R is $C_1$—$C_4$ alkyl;
$R^1$ is hydrogen or $C_1$—$C_4$ alkyl;
$R^2$ is chlorine, bromine, iodine, or methoxy;
$R^3$ and $R^4$ independently are hydrogen, chlorine, bromine, methyl, methoxy, nitro, $CF_3$, or $R^bSO_n$— wherein $R^b$ is $C_1$—$C_4$ alkyl; and n is the integer 2;
$R^5$ is hydrogen or $C_1$—$C_4$ alkyl;
$R^6$ is hydrogen or $C_1$—$C_4$ alkyl;
$R^7$ is hydrogen or $C_1$—$C_4$ alkyl; and
$R^8$ is hydrogen or $C_1$—$C_4$ alkyl;
and their salts.

3. The compounds of Claim 1 wherein:
R is methyl;

$R^1$ is hydrogen or methyl;
$R^2$ is chlorine, bromine, iodine, or methoxy;
$R^3$ and $R^4$ independently are hydrogen, chlorine, bromine, methyl, methoxy, nitro, $CF_3$, or $R^bSO_n$— wherein $R^b$ is $C_1$—$C_4$ alkyl; and n is the integer 2;
$R^5$ is hydrogen or methyl;
$R^6$ is hydrogen or methyl;
$R^7$ is hydrogen or methyl; and
$R^8$ is hydrogen or methyl;
and their salts.

4. The compound of Claim 2 wherein R is methyl, $R^1$ is methyl, $R^2$ is chlorine, $R^3$ is hydrogen, $R^4$ is 4-chlorine, $R^5$ is hydrogen, $R^6$ is hydrogen, $R^7$ is hydrogen and $R^8$ is hydrogen, and its salts.

5. The triethanolammonium salt of the compound of Claim 4.

6. The compound of Claim 2 wherein R is methyl, $R^1$ is methyl, $R^2$ is chlorine, $R^3$ is hydrogen, $R^4$ is 2-$CH_3SO_2$—, $R^5$ is hydrogen, $R^6$ is hydrogen, $R^7$ is hydrogen and $R^8$ is hydrogen and its salts.

7. The method of controlling undesirable vegetation comprising applying to the area where control is desired, an herbicidally effective amount of a compound having the formula

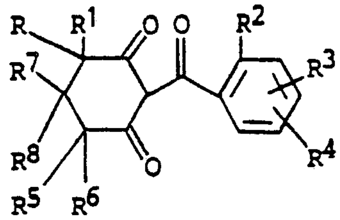

I

wherein
R is $C_1$—$C_6$ alkyl;
$R^1$ is hydrogen, $C_1$—$C_6$ alkyl, or

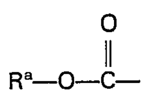

wherein $R^a$ is $C_1$—$C_4$ alkyl or R and $R^1$ together are alkylene having 3 to 6 carbon atoms;
$R^2$ is chlorine, bromine, iodine, or $C_1$—$C_4$ alkoxy;
$R^3$ and $R^4$ independently are
(1) hydrogen;
(2) halogen;
(3) $C_1$—$C_4$ alkyl;
(4) $C_1$—$C_4$ aliphatic alkoxy;
(5) nitro;
(6) $C_1$—$C_4$ haloalkyl; and
(7) $R^bSO_n$— wherein $R^b$ is $C_1$—$C_4$ alkyl; and
n is the integer 0, 1 or 2;
$R^5$ is hydrogen or $C_1$—$C_6$ alkyl; and
$R^6$ is hydrogen or $C_1$—$C_6$ alkyl; and
$R^7$ is hydrogen or $C_1$—$C_6$ alkyl; and
$R^8$ is hydrogen or $C_1$—$C_6$ alkyl;
and their salts.

8. The method of Claim 7 wherein:
R is $C_1$—$C_4$ alkyl;
$R^1$ is hydrogen or $C_1$—$C_4$ alkyl;
$R^2$ is chlorine, bromine, iodine, or methoxy;
$R^3$ and $R^4$ independently are hydrogen, chlorine, bromine, methyl, methoxy, nitro, $CF_3$, or $R^bSO_n$— wherein $R^b$ is $C_1$—$C_4$ alkyl; and n is the integer 2;
$R^5$ is hydrogen or $C_1$—$C_4$ alkyl;
$R^6$ is hydrogen or $C_1$—$C_4$ alkyl;
$R^7$ is hydrogen or $C_1$—$C_4$ alkyl; and
$R^8$ is hydrogen or $C_1$—$C_4$ alkyl;
and their salts.

9. The method of Claim 8 wherein:
R is methyl;
$R^1$ is hydrogen or methyl;
$R^2$ is chlorine, bromine, iodine, or methoxy;
$R^3$ and $R^4$ independently are hydrogen, chlorine, bromine, methyl, methoxy, nitro, $CF_3$, or $R^bSO_n$—

wherein $R^b$ is $C_1$—$C_4$ alkyl; and n is the integer 2;

$R^5$ is hydrogen or methyl;

$R^6$ is hydrogen or methyl;

$R^7$ is hydrogen or methyl; and

$R^8$ is hydrogen or methyl;

and their salts.

10. The compound of Claim 9 wherein R is methyl, $R^1$ is methyl, $R^2$ is chlorine, $R^3$ is hydrogen, $R^4$ is 4-chlorine, $R^5$ is hydrogen, $R^6$ is hydrogen, $R^7$ is hydrogen and $R^8$ is hydrogen, and its salts.

11. The method of Claim 10 wherein the triethanolammonium salt is used.

12. The method of Claim 9 wherein R is methyl, $R^1$ is methyl, $R^2$ is chlorine, $R^3$ is hydrogen, $R^4$ is 2-$CH_3SO_2$—, $R^5$ is hydrogen, $R^6$ is hydrogen, $R^7$ is hydrogen and $R^8$ is hydrogen and its salts.

13. A herbicidal composition comprising as an effective ingredient at least one compound according to one of the claims 1 to 6 together with usual carriers and/or diluents.

**Claims for the Contracting State: AT**

1. A process for preparing a compound having the structural formula I

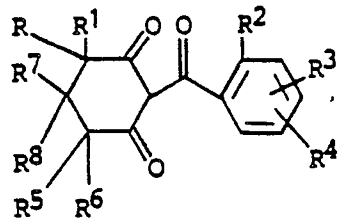

I

wherein

R is $C_1$—$C_6$ alkyl;

$R^1$ is hydrogen, $C_1$—$C_6$ alkyl, or

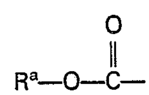

wherein $R^a$ is $C_1$—$C_4$ alkyl or R and $R^1$ together are alkylene having 3 to 6 carbon atoms;

$R^2$ is chlorine, bromine, iodine, or $C_1$—$C_4$ alkoxy;

$R^3$ and $R^4$ independently are

(1) hydrogen;

(2) halogen;

(3) $C_1$—$C_4$ alkyl;

(4) $C_1$—$C_4$ aliphatic alkoxy;

(5) nitro;

(6) $C_1$—$C_4$ haloalkyl; and

(7) $R^bSO_n$— wherein $R^b$ is $C_1$—$C_4$ alkyl; and

n is the integer 0, 1 or 2;

$R^5$ is hydrogen or $C_1$—$C_6$ alkyl; and

$R^6$ is hydrogen or $C_1$—$C_6$ alkyl; and

$R^7$ is hydrogen or $C_1$—$C_6$ alkyl; and

$R^8$ is hydrogen or $C_1$—$C_6$ alkyl;

and their salts, comprising reacting a dione of the general formula II with a substituted benzoyl cyanide of the general formula

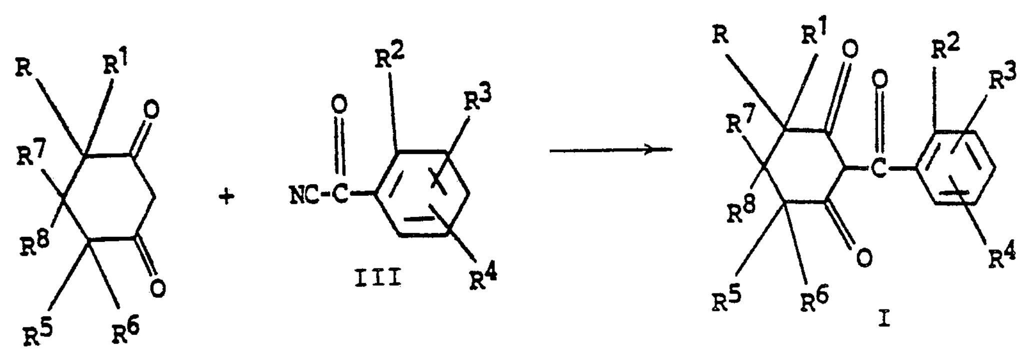

III

I

II

wherein in the general formulas II and III R, $R^1$, $R^2$, $R^3$, $R^4$, $R^5$, $R^6$, $R^7$, $R^8$ have the meaning as given for the compound of formula I.

2. The process of Claim 1 wherein the reaction is carried out in the presence of zinc chloride and triethylamine.

3. The process of Claim 1 wherein:
R is $C_1$—$C_4$ alkyl;
$R^1$ is hydrogen or $C_1$—$C_4$ alkyl;
$R^2$ is chlorine, bromine, iodine, or methoxy;
$R^3$ and $R^4$ independently are hydrogen, chlorine, bromine, methyl, methoxy, nitro, $CF_3$, or $R^bSO_n$— wherein $R^b$ is $C_1$—$C_4$ alkyl; and n is the integer 2;
$R^5$ is hydrogen or $C_1$—$C_4$ alkyl;
$R^6$ is hydrogen or $C_1$—$C_4$ alkyl;
$R^7$ is hydrogen or methyl; and
$R^8$ is hydrogen or methyl;
and their salts.

4. The process of Claim 1 wherein:
R is methyl;
$R^1$ is hydrogen or methyl;
$R^2$ is chlorine, bromine, iodine, or methoxy;
$R^3$ and $R^4$ independently are hydrogen, chlorine, bromine, methyl, methoxy, nitro, $CF_3$, or $R^bSO_n$— wherein $R^b$ is $C_1$—$C_4$ alkyl; and n is the integer 2;
$R^5$ is hydrogen or methyl;
$R^6$ is hydrogen or methyl;
$R^7$ is hydrogen or methyl; and
$R^8$ is hydrogen or methyl;
and their salts.

5. The process of Claim 2 wherein R is methyl, $R^1$ is methyl, $R^2$ is chlorine, $R^3$ is hydrogen, $R^4$ is 4-chlorine, $R^5$ is hydrogen, $R^6$ is hydrogen, $R^7$ is hydrogen and $R^8$ is hydrogen, and their salts.

6. The process of Claim 5 wherein the triethanolammonium salt is used.

7. The process of Claim 2 wherein R is methyl, $R^1$ is methyl, $R^2$ is chlorine, $R^3$ is hydrogen, $R^4$ is 4-$CH_3SO_2$—, $R^5$ is hydrogen, $R^6$ is hydrogen, $R^7$ is hydrogen and $R^8$ is hydrogen and its salts.

8. The method of controlling undesirable vegetation comprising applying to the area where control is desired, an herbicidally effective amount of a compound having the formula

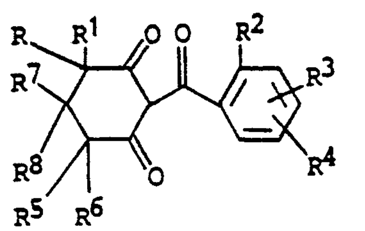

I

wherein
R is $C_1$—$C_6$ alkyl;
$R^1$ is hydrogen, $C_1$—$C_6$ alkyl, or

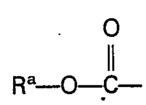

wherein $R^a$ is $C_1$—$C_4$ alkyl or R and $R^1$ together are alkylene having 3 to 6 carbon atoms;
$R^2$ is chlorine, bromine, iodine, or $C_1$—$C_4$ alkoxy;
$R^3$ and $R^4$ independently are
(1) hydrogen;
(2) halogen;
(3) $C_1$—$C_4$ alkyl;
(4) $C_1$—$C_4$ aliphatic alkoxy;
(5) nitro;
(6) $C_1$—$C_4$ haloalkyl; and
(7) $R^bSO_n$— wherein $R^b$ is $C_1$—$C_4$ alkyl; and
n is the integer 0, 1 or 2;
$R^5$ is hydrogen or $C_1$—$C_6$ alkyl; and
$R^6$ is hydrogen or $C_1$—$C_6$ alkyl; and
$R^7$ is hydrogen or $C_1$—$C_6$ alkyl; and
$R^8$ is hydrogen or $C_1$—$C_6$ alkyl;
and their salts.

9. The method of Claim 8 wherein:
R is $C_1$—$C_4$ alkyl;
$R^1$ is hydrogen or $C_1$—$C_4$ alkyl;
$R^2$ is chlorine, bromine, iodine, or methoxy;
$R^3$ and $R^4$ independently are hydrogen, chlorine, bromine, methyl, methoxy, nitro, $CF_3$, or $R^bSO_n$— wherein $R^b$ is $C_1$—$C_4$ alkyl; and n is the integer 2;
$R^5$ is hydrogen or $C_1$—$C_4$ alkyl;
$R^6$ is hydrogen or $C_1$—$C_4$ alkyl;
$R^7$ is hydrogen or $C_1$—$C_4$ alkyl; and
$R^8$ is hydrogen or $C_1$—$C_4$ alkyl;
and their salts.

10. The method of Claim 9 wherein:
R is methyl;
$R^1$ is hydrogen or methyl;
$R^2$ is chlorine, bromine, iodine, or methoxy;
$R^3$ and $R^4$ independently are hydrogen, chlorine, bromine, methyl, methoxy, nitro, $CF_3$, or $R^bSO_n$— wherein $R^b$ is $C_1$—$C_4$ alkyl; and n is the integer 2;
$R^5$ is hydrogen or methyl;
$R^6$ is hydrogen or methyl;
$R^7$ is hydrogen or methyl; and
$R^8$ is hydrogen or methyl;
and their salts.

11. The method of Claim 10 wherein R is methyl, $R^1$ is methyl, $R^2$ is chlorine, $R^3$ is hydrogen, $R^4$ is 4-chlorine, $R^5$ is hydrogen, $R^6$ is hydrogen, $R^7$ is hydrogen and $R^8$ is hydrogen, and its salts.

12. The method of Claim 11 wherein the triethanolammonium salt is used.

13. The method of Claim 10 wherein R is methyl, $R^1$ is methyl, $R^2$ is chlorine, $R^3$ is hydrogen, $R^4$ is 4-$CH_3SO_2$—, $R^5$ is hydrogen, $R^6$ is hydrogen, $R^7$ is hydrogen and $R^8$ is hydrogen and its salts.

**Patentansprüche für die Vertragsstaaten: BE CH DE FR GB IT LI NL SE**

1. Verbindung der Strukturformel

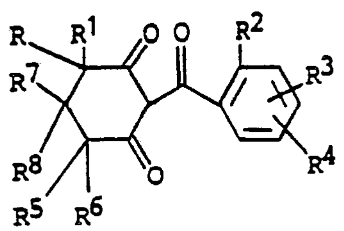

I

worin
R $C_1$—$C_6$-Alkyl bedeutet;
$R^1$ Wasserstoff, $C_1$—$C_6$-Alkyl oder

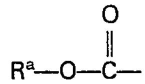

bedeutet,
worin $R^a$ für $C_1$—$C_4$-Alkyl steht oder worin R und $R^1$ zusammen Alkylen mit 3 bis 6 Kohlenstoffatomen bedeuten;
$R^2$ Chlor, Brom, Jod oder $C_1$—$C_4$-Alkoxy bedeutet;
$R^3$ und $R^4$ unabhängig
(1) Wasserstoff;
(2) Halogen;
(3) $C_1$—$C_4$-Alkyl;
(4) $C_1$—$C_4$-aliphatisches Alkoxy;
(5) Nitro;
(6) $C_1$—$C_4$-Haloalkyl; und
(7) $R^bSO_n$—, worin $R^b$ für $C_1$—$C_4$-Alkyl und n für eine ganze Zahl von 0, 1 oder 2 stehen, bedeuten;
$R^5$ Wasserstoff oder $C_1$—$C_6$-Alkyl bedeutet; und
$R^6$ Wasserstoff oder $C_1$—$C_6$-Alkyl bedeutet; und
$R^7$ Wasserstoff oder $C_1$—$C_6$-Alkyl bedeutet; und
$R^8$ Wasserstoff oder $C_1$—$C_6$-Alkyl bedeutet;
und ihre Salze.

2. Verbindung nach Anspruch 1, worin

R $C_1$—$C_4$-Alkyl bedeutet;

$R^1$ Wasserstoff oder $C_1$—$C_4$-Alkyl bedeutet;

$R^2$ Chlor, Brom, Jod oder Methoxy bedeutet;

$R^3$ und $R^4$ unabhängig Wasserstoff, Chlor, Brom, Methyl, Methoxy, Nitro, $CF_3$ oder $R^bSO_n$— bedeuten, worin $R^b$ für $C_1$—$C_4$-Alkyl und n für eine ganze Zahl von 2 stehen;

$R^5$ Wasserstoff oder $C_1$—$C_4$-Alkyl bedeutet;

$R^6$ Wasserstoff oder $C_1$—$C_4$-Alkyl bedeutet;

$R^7$ Wasserstoff oder $C_1$—$C_4$-Alkyl bedeutet; und

$R^8$ Wasserstoff oder $C_1$—$C_4$-Alkyl bedeutet;

und ihre Salze.

3. Verbindung nach Anspruch 1, worin

R Methyl bedeutet;

$R^1$ Wasserstoff oder Methyl bedeutet;

$R^2$ Chlor, Brom, Jod oder Methoxy bedeutet;

$R^3$ und $R^4$ unabhängig Wasserstoff, Chlor, Brom, Methyl, Methoxy, Nitro, $CF_3$ oder $R^bSO_n$— bedeuten, worin $R^b$ $C_1$—$C_4$-Alkyl bedeutet und n für eine ganze Zahl von 2 bedeutet;

$R^5$ Wasserstoff oder Methyl bedeutet;

$R^6$ Wasserstoff oder Methyl bedeutet;

$R^7$ Wasserstoff oder Methyl bedeutet; und

$R^8$ Wasserstoff oder Methyl bedeutet;

und ihre Salze.

4. Verbindung nach Anspruch 2, worin R Methyl, $R^1$ Methyl, $R^2$ Chlor, $R^3$ Wasserstoff, $R^4$ 4-Chlor, $R^5$ Wasserstoff, $R^6$ Wasserstoff, $R^7$ Wasserstoff bedeuten, und ihre Salze.

5. Das Triethanolammoniumsalz der Verbindung nach Anspruch 4.

6. Verbindung nach Anspruch 2, worin R Methyl, $R^1$ Methyl, $R^2$ Chlor, $R^3$ Wasserstoff, $R^4$ 4-$CH_3SO_2$—, $R^5$ Wasserstoff, $R^6$ Wasserstoff, $R^7$ Wasserstoff und $R^8$ Wasserstoff bedeuten, und ihre Salze.

7. Verfahren zur Kontrolle unerwünschter Vegetation, dadurch gekennzeichnet, daß man auf die Fläche, wo eine Kontrolle erfolgen soll, eine herbizid wirksame Menge einer Verbindung der Formel

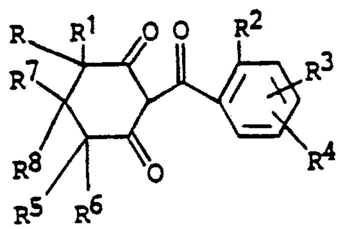

I

anwendet, worin

R $C_1$—$C_6$-Alkyl bedeutet;

$R^1$ Wasserstoff, $C_1$—$C_6$-Alkyl oder

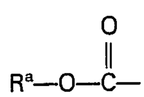

bedeutet,

worin $R^a$ für $C_1$—$C_4$-Alkyl steht oder worin R und $R^1$ zusammen Alkylen mit 3 bis 6 Kohlenstoffatomen bedeuten;

$R^2$ Chlor, Brom, Jod oder $C_1$—$C_4$-Alkoxy bedeutet;

$R^3$ und $R^4$ unabhängig

(1) Wasserstoff;

(2) Halogen;

(3) $C_1$—$C_4$-Alkyl;

(4) $C_1$—$C_4$-aliphatisches Alkoxy;

(5) Nitro;

(6) $C_1$—$C_4$-Haloalkyl; und

(7) $R^bSO_n$—, worin $R^b$ für $C_1$—$C_4$-Alkyl und n für eine ganze Zahl von 0, 1 oder 2 stehen, bedeuten;

$R^5$ Wasserstoff oder $C_1$—$C_6$-Alkyl bedeutet; und

$R^6$ Wasserstoff oder $C_1$—$C_6$-Alkyl bedeutet; und

$R^7$ Wasserstoff oder $C_1$—$C_6$-Alkyl bedeutet; und

$R^8$ Wasserstoff oder $C_1$—$C_6$-Alkyl bedeutet;

und ihr Salze.

8. Verfahren nach Anspruch 7, worin

R $C_1$—$C_4$-Alkyl bedeutet;
$R^1$ Wasserstoff oder $C_1$—$C_4$-Alkyl bedeutet;
$R^2$ Chlor, Brom, Jod oder Methoxy bedeutet;
$R^3$ und $R^4$ unabhängig Wasserstoff, Chlor, Brom, Methyl, Methoxy, Nitro, $CF_3$ oder $R^bSO_n$— bedeuten, worin $R^b$ $C_1$—$C_4$-Alkyl und n eine ganze Zahl von 2 bedeuten;
$R^5$ Wasserstoff oder $C_1$—$C_4$-Alkyl bedeutet;
$R^6$ Wasserstoff oder $C_1$—$C_4$-Alkyl bedeutet;
$R^7$ Wasserstoff oder $C_1$—$C_4$-Alkyl bedeutet; und
$R^8$ Wasserstoff oder $C_1$—$C_4$-Alkyl bedeutet;
und ihre Salze.

9. Verfahren nach Anspruch 8, worin
R Methyl bedeutet;
$R^1$ Wasserstoff oder Methyl bedeutet;
$R^2$ Chlor, Brom, Jod oder Methoxy bedeutet;
$R^3$ und $R^4$ unabhängig Wasserstoff, Chlor, Brom, Methyl, Methoxy, Nitro, $CF_3$ oder $R^bSO_n$— bedeuten, worin $R^b$ $C_1$—$C_4$-Alkyl und n für eine ganze Zahl von 2 bedeuten;
$R^5$ Wasserstoff oder Methyl bedeutet;
$R^6$ Wasserstoff oder Methyl bedeutet;
$R^7$ Wasserstoff oder Methyl bedeutet; und
$R^8$ Wasserstoff oder Methyl bedeutet;
und ihre Salze.

10. Verfahren nach Anspruch 9, worin R Methyl, $R^1$ Methyl, $R^2$ Chlor, $R^3$ Wasserstoff, $R^4$ 4-Chlor, $R^5$ Wasserstoff, $R^6$ Wasserstoff, $R^7$ Wasserstoff bedeuten, und ihre Salze.

11. Verfahren nach Anspruch 10, bei dem das Triethanolammoniumsalz verwendet wird.

12. Verfahren nach Anspruch 9, worin R Methyl, $R^1$ Methyl, $R^2$ Chlor, $R^3$ Wasserstoff, $R^4$ 4-$CH_3SO_2$—, $R^5$ Wasserstoff, $R^6$ Wasserstoff, $R^7$ Wasserstoff und $R^8$ Wasserstoff bedeuten, und ihre Salze.

13. Herbizides Präparat, dadurch gekennzeichnet, daß es als wirksamen Bestandteil mindestens eine Verbindung nach einem der Ansprüche 1 bis 6 zusammen mit üblichen Trägerstoffen und/oder Verdünnungsmittel enthält.

**Patentansprüche für den Vertragsstaat: AT**

1. Verfahren zur Herstellung einer Verbindung der Strukturformel I

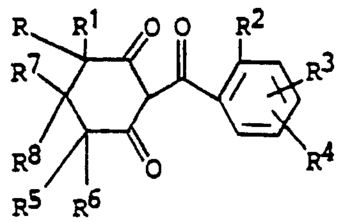

I

worin
R $C_1$—$C_6$-Alkyl bedeutet;
$R^1$ Wasserstoff, $C_1$—$C_6$-Alkyl oder

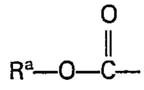

bedeutet,
worin $R^a$ $C_1$—$C_4$-Alkyl steht oder worin R und $R^1$ zusammen Alkylen mit 3 bis 6 Kohlenstoffatomen bedeuten;
$R^2$ Chlor, Brom, Jod oder $C_1$—$C_4$-Alkoxy bedeutet;
$R^3$ und $R^4$ unabhängig
(1) Wasserstoff;
(2) Halogen;
(3) $C_1$—$C_4$-Alkyl;
(4) $C_1$—$C_4$-aliphatisches Alkoxy;
(5) Nitro;
(6) $C_1$—$C_4$-Haloalkyl; und
(7) $R^bSO_n$—, bedeutet, worin $R^b$ $C_1$—$C_4$-Alkyl und n eine ganze Zahl von 0, 1 oder 2 bedeuten;
$R^5$ Wasserstoff oder $C_1$—$C_6$-Alkyl bedeutet; und
$R^6$ Wasserstoff oder $C_1$—$C_6$-Alkyl bedeutet; und
$R^7$ Wasserstoff oder $C_1$—$C_6$-Alkyl bedeutet; und

$R^8$ Wasserstoff oder $C_1$—$C_6$-Alkyl bedeutet;
und ihr Salze, dadurch gekennzeichnet, daß man ein Dion der allgemeinen Formel II mit einem substituierten Benzoylcyanid der allgemeinen Formel

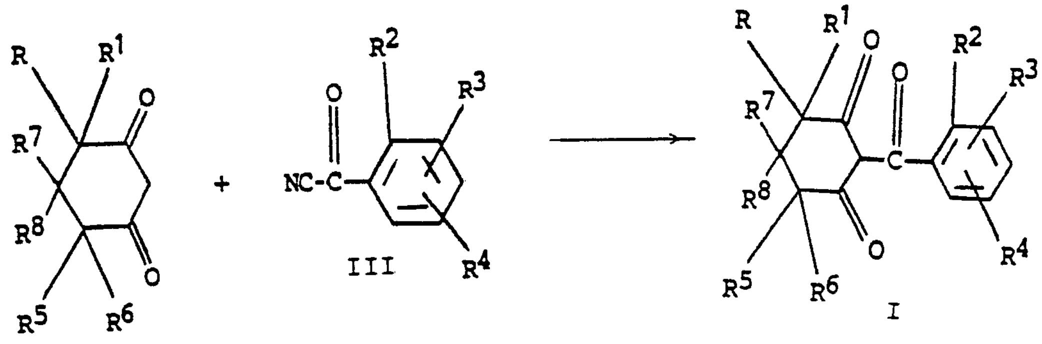

II III I

umsetzt, worin in den allgemeinen Formeln II und II, R, $R^1$, $R^2$, $R^3$, $R^4$, $R^5$, $R^6$, $R^7$, $R^8$ die Bedeutungen besitzen, wie sie für die Verbindung der Formel I angegeben wurden.

2. Verfahren nach Anspruch 1, dadurch gekennzeichnet, daß die Reaktion in Anwesenheit von Zinkchlorid und Triethylamin durchgeführt wird.

3. Verfahren nach Anspruch 1, bei dem
R $C_1$—$C_4$-Alkyl bedeutet;
$R^1$ Wasserstoff oder $C_1$—$C_4$-Alkyl bedeutet;
$R^2$ Chlor, Brom, Jod oder Methoxy bedeutet;
$R^3$ und $R^4$ unabhängig Wasserstoff, Chlor, Brom, Methyl, Methoxy, Nitro, $CF_3$ oder $R^bSO_n$— bedeuten, worin $R^b$ $C_1$—$C_4$-Alkyl und n eine ganze Zahl von 2 bedeuten;
$R^5$ Wasserstoff oder $C_1$—$C_4$-Alkyl bedeutet;
$R^6$ Wasserstoff oder $C_1$—$C_4$-Alkyl bedeutet;
$R^7$ Wasserstoff oder methyl bedeutet; und
$R^8$ Wasserstoff oder methyl bedeutet;
und ihre Salze.

4. Verfahren nach Anspruch 1, bei dem
R Methyl bedeutet;
$R^1$ Wasserstoff oder Methyl bedeutet;
$R^2$ Chlor, Brom, Jod oder Methoxy bedeutet;
$R^3$ und $R^4$ unabhängig Wasserstoff, Chlor, Brom, Methyl, Methoxy, Nitro, $CF_3$ oder $R^bSO_n$— bedeuten, worin $R^b$ $C_1$—$C_4$-Alkyl und n eine ganze Zahl von 2 bedeuten;
$R^5$ Wasserstoff oder Methyl bedeutet;
$R^6$ Wasserstoff oder Methyl bedeutet;
$R^7$ Wasserstoff oder Methyl bedeutet; und
$R^8$ Wasserstoff oder Methyl bedeutet;
und ihre Salze.

5. Verfahren nach Anspruch 2, worin R Methyl, $R^1$ Methyl, $R^2$ Chlor, $R^3$ Wasserstoff, $R^4$ 4-Chlor, $R^5$ Wasserstoff, $R^6$ Wasserstoff, $R^7$ Wasserstoff bedeuten, und ihre Salze.

6. Verfahren nach Anspruch 5, bei dem das Triethanolammoniumsalz verwendet wird.

7. Verfahren nach Anspruch 2, bei dem R Methyl, $R^1$ Methyl, $R^2$ Chlor, $R^3$ Wasserstoff, $R^4$ 4-$CH_3SO_2$—, $R^5$ Wasserstoff, $R^6$ Wasserstoff, $R^7$ Wasserstoff und $R^8$ Wasserstoff bedeuten, und ihre Salze.

8. Verfahren zur Kontrolle unerwünschter Vegetation, dadurch gekennzeichnet, daß man auf die Fläche, wo eine Kontrolle erfolgen soll, eine herbizid wirksame Menge einer Verbindung der Formel

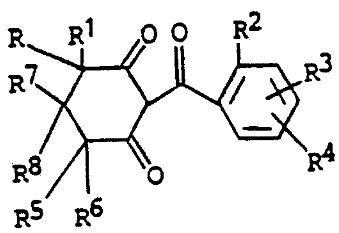

I

anwendet, worin
R $C_1$—$C_6$-Alkyl bedeutet;
$R^1$ Wasserstoff, $C_1$—$C_6$-Alkyl oder

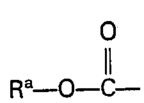

bedeutet,
worin $R^a$ $C_1$—$C_4$-Alkyl bedeutet oder worin R und $R^1$ zusammen Alkylen mit 3 bis 6 Kohlenstoffatomen bedeuten;

$R^2$ Chlor, Brom, Jod oder $C_1$—$C_4$-Alkoxy bedeutet;

$R^3$ und $R^4$ unabhängig
(1) Wasserstoff;
(2) Halogen;
(3) $C_1$—$C_4$-Alkyl;
(4) $C_1$—$C_4$-aliphatisches Alkoxy;
(5) Nitro;
(6) $C_1$—$C_4$-Haloalkyl; und
(7) $R^bSO_n$— bedeuten, worin $R^b$ $C_1$—$C_4$-Alkyl und n eine ganze Zahl von 0, 1 oder 2 stehen, bedeuten;

$R^5$ Wasserstoff oder $C_1$—$C_6$-Alkyl; und
$R^6$ Wasserstoff oder $C_1$—$C_6$-Alkyl; und
$R^7$ Wasserstoff oder $C_1$—$C_6$-Alkyl; und
$R^8$ Wasserstoff oder $C_1$—$C_6$-Alkyl bedeuten;
und ihre Salze.

9. Verfahren nach Anspruch 8, bei dem
R $C_1$—$C_4$-Alkyl bedeutet;
$R^1$ Wasserstoff oder $C_1$—$C_4$-Alkyl bedeutet;
$R^2$ Chlor, Brom, Jod oder Methoxy bedeutet;
$R^3$ und $R^4$ unabhängig Wasserstoff, Chlor, Brom, Methyl, Methoxy, Nitro, $CF_3$ oder $R^bSO_n$— bedeuten, worin $R^b$ $C_1$—$C_4$-Alkyl und n eine ganze Zahl von 2 bedeuten;
$R^5$ Wasserstoff oder $C_1$—$C_4$-Alkyl bedeutet;
$R^6$ Wasserstoff oder $C_1$—$C_4$-Alkyl bedeutet;
$R^7$ Wasserstoff oder $C_1$—$C_4$-Alkyl bedeutet; und
$R^8$ Wasserstoff oder $C_1$—$C_4$-Alkyl bedeutet;
und ihre Salze.

10. Verfahren nach Anspruch 9, bei dem
R Methyl bedeutet;
$R^1$ Wasserstoff oder Methyl bedeutet;
$R^2$ Chlor, Brom, Jod oder Methoxy bedeutet;
$R^3$ und $R^4$ unabhängig Wasserstoff, Chlor, Brom, Methyl, Methoxy, Nitro, $CF_3$ oder $R^bSO_n$— bedeuten, worin $R^b$ $C_1$—$C_4$-Alkyl und n für eine ganze Zahl von 2 bedeuten;
$R^5$ Wasserstoff oder Methyl bedeutet;
$R^6$ Wasserstoff oder Methyl bedeutet;
$R^7$ Wasserstoff oder Methyl bedeutet; und
$R^8$ Wasserstoff oder Methyl bedeutet;
und ihre Salze.

11. Verfahren nach Anspruch 10, bei dem R Methyl, $R^1$ Methyl, $R^2$ Chlor, $R^3$ Wasserstoff, $R^4$ 4-Chlor, $R^5$ Wasserstoff, $R^6$ Wasserstoff, $R^7$ Wasserstoff und $R^5$ Wasserstoff bedeuten, und ihre Salze.

12. Verfahren nach Anspruch 11, bei dem das Triethanolammoniumsalz verwendet wird.

13. Verfahren nach Anspruch 10, bei dem R Methyl, $R^1$ Methyl, $R^2$ Chlor, $R^3$ Wasserstoff, $R^4$ 4-$CH_3SO_2$—, $R^5$ Wasserstoff, $R^6$ Wasserstoff, $R^7$ Wasserstoff und $R^8$ Wasserstoff bedeuten, und ihre Salze.

**Revendications pour les Etats contractants: BE CH DE FR GB IT LI NL SE**

1. Un dérivé de formule structurelle:

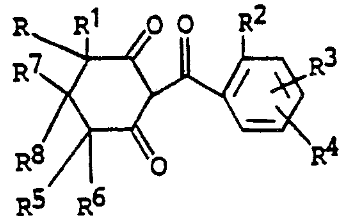

I

dans laquelle:
R représente un radical alkyle en $C_1$ à $C_6$;
$R^1$ représente un atome d'hydrogène, un radical alkyle en $C_1$ à $C_6$ ou

$$R^a—O—\overset{O}{\overset{\|}{C}}—$$

dans laquelle $R^a$ représente un radical alkyle en $C_1$ à $C_4$, ou R et $R^1$ forment ensemble un radical alkylène renfermant de 3 à 6 atomes de carbone;

$R^2$ représente un atome de chlore, de brome, d'iode ou un radical alkoxy en $C_1$ à $C_4$;

$R^3$ et $R^4$ représente indépendamment:

(1) un atome d'hydrogène;

(2) un atome d'halogène;

(3) un radical alkyle en $C_1$ à $C_4$;

(4) un radical alkoxy aliphatique en $C_1$ à $C_4$;

(5) un groupe nitro;

(6) un radical haloalkyle en $C_1$ à $C_4$; et

(7) $R^bSO_n$— dans lequel $R^b$ représente un radical alkyle en $C_1$ à $C_4$ et n est un entier égal à 0, 1 ou 2;

$R^5$ représente un atome d'hydrogène ou un radical alkyle en $C_1$ à $C_6$; et

$R^6$ représente un atome d'hydrogène ou un radical alkyle en $C_1$ à $C_6$; et

$R^7$ représente un atome d'hydrogène ou un radical alkyle en $C_1$ à $C_6$; et

$R^8$ représente un atome d'hydrogène ou un radical alkyle en $C_1$ à $C_6$; et

et leurs sels.

2. Les dérivés selon la revendication 1, dans lesquels:

R représente un radical alkyle en $C_1$ à $C_4$;

$R^1$ représente un atome d'hydrogène ou un radical alkyle en $C_1$ à $C_4$;

$R^2$ représente un atome de chlore, de brome, d'iode ou un groupe méthoxy;

$R^3$ et $R^4$ représentent indépendamment un atome d'hydrogène, de chlore, de brome, un radical méthyle, méthoxy, un groupe nitro, $CF_3$ ou $R^bSO_n$— dans lequel $R^b$ représente un radical alkyle en $C_1$ à $C_4$ et n est égal à 2;

$R^5$ représente un atome d'hydrogène ou un radical alkyle en $C_1$ à $C_4$;

$R^6$ représente un atome d'hydrogène ou un radical alkyle en $C_1$ à $C_4$;

$R^7$ représente un atome d'hydrogène ou un radical alkyle en $C_1$ à $C_4$;

$R^8$ représente un atome d'hydrogène ou un radical alkyle en $C_1$ à $C_4$;

et leurs sels.

3. Les dérivés selon la revendication 1, dans lesquels:

R représente un radical méthyle;

$R^1$ représente un atome d'hydrogène ou un radical méthyle;

$R^2$ représente un atome de chlore, de brome, d'iode ou un groupe méthoxy;

$R^3$ et $R^4$ représentent indépendamment un atome d'hydrogène, de chlore, de brome, un radical méthyle, méthoxy, un groupe nitro, $CF_3$ ou $R^bSO_n$— dans lequel $R^b$ représente un radical alkyle en $C_1$ à $C_4$ et n est égal à 2;

$R^5$ représente un atome d'hydrogène ou un radical méthyle;

$R^6$ représente un atome d'hydrogène ou un radical méthyle;

$R^7$ représente un atome d'hydrogène ou un radical méthyle;

$R^8$ représente un atome d'hydrogène ou un radical méthyle;

et leurs sels.

4. Les dérivés selon la revendication 2, dans lesquels R représente le radical méthyle, $R^1$ représente le radical méthyle, $R^2$ représente un atome de chlore, $R^3$ représente un atome d'hydrogène, $R^4$ représente un atome de chlore en position 4, $R^5$ représente un atome d'hydrogène, $R^6$ représente un atome d'hydrogène, $R^7$ représente un atome d'hydrogène, $R^8$ représente un atome hydrogène, et leurs sels.

5. Le sel de triéthanolammonium du dérivé selon la revendication 4.

6. Le dérivé selon la revendication 2, dans lequel R représente le radical méthyle, $R^1$ représente la radical méthyle, $R^2$ représente un atome de chlore, $R^3$ représente un atome d'hydrogène, $R^4$ représente 4-$CH_3SO_2$—, $R^5$ représente un atome d'hydrogène, $R^6$ représente un atome d'hydrogène, $R^7$ représente un atome d'hydrogène, $R^8$ représente un atome d'hydrogène, et ses sels.

7. Le procédé de contrôle d'une végétation indésirable consistant à appliquer à la zone que l'on souhaite contrôler une quantité efficace en tant qu'herbicide d'un dérivé de formule:

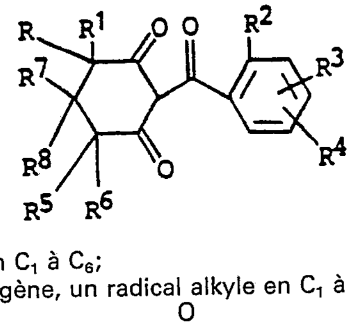

I

dans laquelle:

R représente un radical alkyle en $C_1$ à $C_6$;

$R^1$ représente un atome d'hydrogène, un radical alkyle en $C_1$ à $C_6$ ou

$$R^a{-}O{-}\overset{\overset{\displaystyle O}{\|}}{C}{-}$$

dans laquelle $R^a$ représente un radical alkyle en $C_1$ à $C_4$, ou R et $R^1$ forment ensemble un radical alkylène renfermant de 3 à 6 atomes de carbone;

$R^2$ représente un atome de chlore, de brome, d'iode ou un radical alkoxy en $C_1$ à $C_4$;

$R^3$ et $R^4$ représente indépendamment:

(1) un atome d'hydrogène;

(2) un atome d'halogène;

(3) un radical alkyle en $C_1$ à $C_4$;

(4) un radical alkoxy aliphatique en $C_1$ à $C_4$;

(5) un groupe nitro;

(6) un radical haloalkyle en $C_1$ à $C_4$; et

(7) $R^bSO_n$— dans lequel $R^b$ représente un radical alkyle en $C_1$ à $C_4$ et n est un entier égal à 0, 1 ou 2;

$R^5$ représente un atome d'hydrogène ou un radical alkyle en $C_1$ à $C_6$; et

$R^6$ représente un atome d'hydrogène ou un radical alkyle en $C_1$ à $C_6$; et

$R^7$ représente un atome d'hydrogène ou un radical alkyle en $C_1$ à $C_6$; et

$R^8$ représente un atome d'hydrogène ou un radical alkyle en $C_1$ à $C_6$; et

et leurs sels.

8. Le procédé selon la revendication 7, dans lequel:

R représente un radical alkyle $C_1$ à $C_4$;

$R^1$ représente un atome d'hydrogène ou un radical alkyle en $C_1$ à $C_4$;

$R^2$ représente un atome de chlore, de brome, d'iode ou un groupe méthoxy;

$R^3$ et $R^4$ représentent indépendamment un atome d'hydrogène, de chlore, de brome, un radical méthyle, méthoxy, un groupe nitro, $CF_3$ ou $R^bSO_n$— dans lequel $R^b$ représente un radical alkyle en $C_1$ à $C_4$ et n est égal à 2;

$R^5$ représente un atome d'hydrogène ou un radical alkyle en $C_1$ à $C_4$;

$R^6$ représente un atome d'hydrogène ou un radical alkyle en $C_1$ à $C_4$;

$R^7$ représente un atome d'hydrogène ou un radical alkyle en $C_1$ à $C_4$;

$R^8$ représente un atome d'hydrogène ou un radical alkyle en $C_1$ à $C_4$;

et leurs sels.

9. Le procédé selon la revendication 8, dans lequel:

R représente un radical méthyle;

$R^1$ représente un atome d'hydrogène ou un radical méthyle;

$R^2$ représente un atome de chlore, de brome, d'iode ou un groupe méthoxy;

$R^3$ et $R^4$ représentent indépendamment un atome d'hydrogène, de chlore, de brome, un radical méthyle, méthoxy, un groupe nitro, $CF_3$ ou $R^bSO_n$— dans lequel $R^b$ représente un radical alkyle en $C_1$ à $C_4$ et n est égal à 2;

$R^5$ représente un atome d'hydrogène ou un radical méthyle;

$R^6$ représente un atome d'hydrogène ou un radical méthyle;

$R^7$ représente un atome d'hydrogène ou un radical méthyle;

$R^8$ représente un atome d'hydrogène ou un radical méthyle;

et leurs sels.

10. Le procédé selon la revendication 9, dans lequel R représente le radical méthyle, $R^1$ représente le radical méthyle, $R^2$ représente un atome de chlore, $R^3$ représente un atome d'hydrogène, $R^4$ représente un atome de chlore en position 4, $R^5$ représente un atome d'hydrogène, $R^6$ représente un atome d'hydrogène, $R^7$ représente un atome d'hydrogène, $R^8$ représente un atome d'hydrogène, et ses sels.

11. Le procédé selon la revendication 10, dans lequel utilise le sel de triéthanolammonium.

12. Le procédé selon la revendication 9, dans lequel R représente le radical méthyle, $R^1$ représente la radical méthyle, $R^2$ représente un atome de chlore, $R^3$ représente un atome d'hydrogène, $R^4$ représente 4-$CH_3SO_2$—, $R^5$ représente un atome d'hydrogène, $R^6$ représente un atome d'hydrogène, $R^7$ représente un atome d'hydrogène, $R^8$ représente un atome d'hydrogène, et ses sels.

13. Une composition herbicide comprenant comme ingrédient efficace au moins un dérivé selon l'une quelconque des revendications 1 à 6 ci-dessus en même temps que des supports et/ou diluants classiques.

**Revendications pour l'Etat contractant: AT**

1. Un procédé de préparation d'un dérivé de formule structurelle I:

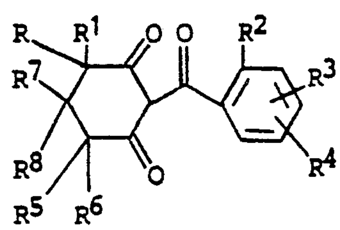

I

dans laquelle:

R représente un radical alkyle en $C_1$ à $C_6$;

$R^1$ représente un atome d'hydrogène, un radical alkyle en $C_1$ à $C_6$ ou

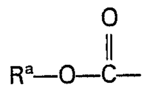

dans laquelle $R^a$ représente un radical alkyle en $C_1$ à $C_4$, ou R et $R^1$ forment ensemble un radical alkylène renfermant de 3 à 6 atomes de carbone;

$R^2$ représente un atome de chlore, de brome, d'iode ou un radical alkoxy en $C_1$ à $C_4$;

$R^3$ et $R^4$ représente indépendamment:

(1) un atome d'hydrogène;
(2) un atome d'halogène;
(3) un radical alkyle en $C_1$ à $C_4$;
(4) un radical alkoxy aliphatique en $C_1$ à $C_4$;
(5) un groupe nitro;
(6) un radical haloalkyle en $C_1$ à $C_4$; et
(7) $R^bSO_n$— dans lequel $R^b$ représente un radical alkyle en $C_1$ à $C_4$ et n est un entier égal à 0, 1 ou 2;

$R^5$ représente un atome d'hydrogène ou un radical alkyle en $C_1$ à $C_6$; et

$R^6$ représente un atome d'hydrogène ou un radical alkyle en $C_1$ à $C_6$; et

$R^7$ représente un atome d'hydrogène ou un radical alkyle en $C_1$ à $C_6$; et

$R^8$ représente un atome d'hydrogène ou un radical alkyle en $C_1$ à $C_6$; et

et leurs sels, consistant à faire réagir une dione de formule générale II avec un cyanure de benzoyle substitué de formule générale:

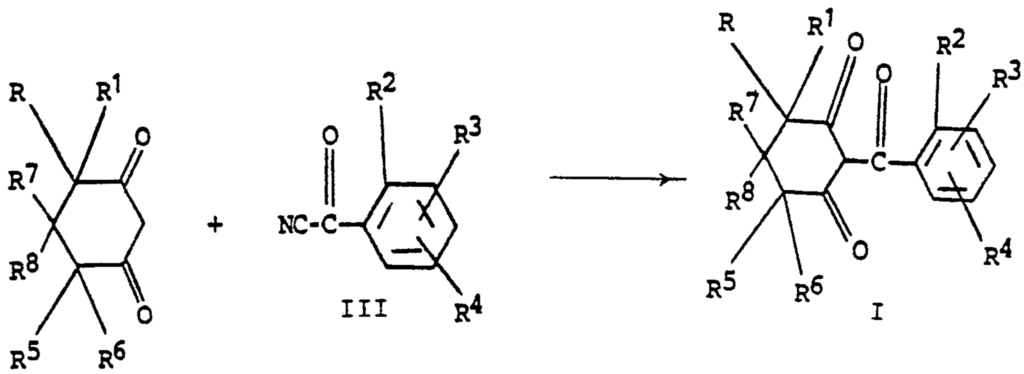

+

III

I

II

dans laquelle, dans les formules générales II et III, $R^1$, $R^2$, $R^3$, $R^4$, $R^5$, $R^6$, $R^7$ et $R^8$ ont les significations indiquées pour le dérivé de formule I.

2. Le procédé selon la revendication 1, dans lequel on met la réaction en oeuvre en présence de chlorure de zinc et de triéthylamine.

3. Le procédé selon la revendication 1, dans lequel:

R représente un radical alkyle $C_1$ en à $C_4$;

$R^1$ représente un atome d'hydrogène ou un radical alkyle en $C_1$ à $C_4$;

$R^2$ représente un atome de chlore, de brome, d'iode ou un groupe méthoxy;

$R^3$ et $R^4$ représentent indépendamment un atome d'hydrogène, de chlore, de brome, un radical méthyle, méthoxy, un groupe nitro, $CF_3$ ou $R^bSO_n$— dans lequel $R^b$ représente un radical alkyle en $C_1$ à $C_4$ et n est égal à 2;

$R^5$ représente un atome d'hydrogène ou un radical alkyle en $C_1$ à $C_4$;

$R^6$ représente un atome d'hydrogène ou un radical alkyle en $C_1$ à $C_4$;

$R^7$ représente un atome d'hydrogène ou un radical methyl;

$R^8$ représente un atome d'hydrogène ou un radical methyl;

et leurs sels.

4. Le procédé selon la revendication 1, dans lequel:

R représente un radical méthyle;

$R^1$ représente un atome d'hydrogène ou un radical méthyle;

$R^2$ représente un atome de chlore, de brome, d'iode ou un groupe méthoxy;

$R^3$ et $R^4$ représentent indépendamment un atome d'hydrogène, de chlore, de brome, un radical méthyle, méthoxy, un groupe nitro, $CF_3$ ou $R^bSO_n$— dans lequel $R^b$ représente un radical alkyle en $C_1$ à $C_4$ et n est égal à 2;

$R^5$ représente un atome d'hydrogène ou un radical méthyle;
$R^6$ représente un atome d'hydrogène ou un radical méthyle;
$R^7$ représente un atome d'hydrogène ou un radical méthyle;
$R^8$ représente un atome d'hydrogène ou un radical méthyle;
et leurs sels.

5. Le procédé selon la revendication 2, dans lequel R représente le radical méthyle, $R^1$ représente le radical méthyle, $R^2$ représente un atome de chlore, $R^3$ représente un atome d'hydrogène, $R^4$ représente un atome de chlore en position 4, $R^5$ représente un atome d'hydrogène, $R^6$ représente un atome d'hydrogène, $R^7$ représente un atome d'hydrogène, $R^8$ représente un atome hydrogène, et leurs sels.

6. Le procédé selon la revendication 5, dans lequel on utilise le sel de triéthanolammonium.

7. Le procédé selon la revendication 2, dans lequel R représente le radical méthyle, $R^1$ représente la radical méthyle, $R^2$ représente un atome de chlore, $R^3$ représente un atome d'hydrogène, $R^4$ représente 4-$CH_3SO_2$—, $R^5$ représente un atome d'hydrogène, $R^6$ représente un atome d'hydrogène, $R^7$ représente un atome d'hydrogène, $R^8$ représente un atome d'hydrogène, et ses sels.

8. Le procédé de contrôle d'une végétation indésirable consistant à appliquer à la zone que l'on désire contrôler une quantité efficace en tant qu'herbicide d'un dérivé de formule:

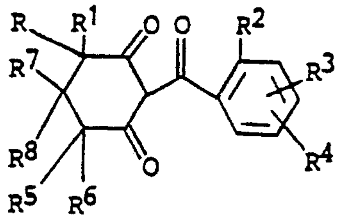

I

dans laquelle:
R représente un radical alkyle en $C_1$ à $C_6$;
$R^1$ représente un atome d'hydrogène, un radical alkyle en $C_1$ à $C_6$ ou

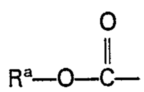

dans laquelle $R^a$ représente un radical alkyle en $C_1$ à $C_4$, ou R et $R^1$ forment ensemble un radical alkylène renfermant de 3 à 6 atomes de carbone;
$R^2$ représente un atome de chlore, de brome, d'iode ou un radical alkoxy en $C_1$ à $C_4$;
$R^3$ et $R^4$ représentent indépendamment:
(1) un atome d'hydrogène;
(2) un atome d'halogène;
(3) un radical alkyle en $C_1$ à $C_4$;
(4) un radical alkoxy aliphatique en $C_1$ à $C_4$;
(5) un groupe nitro;
(6) un radical haloalkyle en $C_1$ à $C_4$; et
(7) $R^bSO_n$— dans lequel $R^b$ représente un radical alkyle en $C_1$ à $C_4$ et n est un entier égal à 0, 1 ou 2;
$R^5$ représente un atome d'hydrogène ou un radical alkyle en $C_1$ à $C_6$; et
$R^6$ représente un atome d'hydrogène ou un radical alkyle en $C_1$ à $C_6$; et
$R^7$ représente un atome d'hydrogène ou un radical alkyle en $C_1$ à $C_6$; et
$R^8$ représente un atome d'hydrogène ou un radical alkyle en $C_1$ à $C_6$; et
et leurs sels.

9. Le procédé selon la revendication 8, dans lequel:
R représente un radical alkyle en $C_1$ à $C_4$;
$R^1$ représente un atome d'hydrogène ou un radical alkyle en $C_1$ à $C_4$;
$R^2$ représente un atome de chlore, de brome, d'iode ou un groupe méthoxy;
$R^3$ et $R^4$ représentent indépendamment un atome d'hydrogène, de chlore, de brome, un radical méthyle, méthoxy, un groupe nitro, $CF_3$ ou $R^bSO_n$— dans lequel $R^b$ représente un radical alkyle en $C_1$ à $C_4$ et n est égal à 2;
$R^5$ représente un atome d'hydrogène ou un radical alkyle en $C_1$ à $C_4$;
$R^6$ représente un atome d'hydrogène ou un radical alkyle en $C_1$ à $C_4$;
$R^7$ représente un atome d'hydrogène ou un radical alkyle en $C_1$ à $C_4$;
$R^8$ représente un atome d'hydrogène ou un radical alkyle en $C_1$ à $C_4$;
et leurs sels.

10. Le procédé selon la revendication 9, dans lequel:
R représente le radical méthyle;
$R^1$ représente un atome d'hydrogène ou un radical méthyle;
$R^2$ représente un atome de chlore, de brome, d'iode ou un groupe méthoxy;

$R^3$ et $R^4$ représentent indépendamment un atome d'hydrogène, de chlore, de brome, un radical méthyle, méthoxy, un groupe nitro, $CF_3$ ou $R^bSO_n$— dans lequel $R^b$ représente un radical alkyle en $C_1$ à $C_4$ et n est égal à 2;

$R^5$ représente un atome d'hydrogène ou le radical méthyle;

$R^6$ représente un atome d'hydrogène ou le radical méthyle;

$R^7$ représente un atome d'hydrogène ou le radical méthyle;

$R^8$ représente un atome d'hydrogène ou le radical méthyle;

et leurs sels.

11. Le procédé selon la revendication 10, dans lequel R représente le radical méthyle, $R^1$ représente le radical méthyle, $R^2$ représente un atome de chlore, $R^3$ représente un atome d'hydrogène, $R^4$ représente un atome de chlore en position 4, $R^5$ représente un atome d'hydrogène, $R^6$ représente un atome d'hydrogène, $R^7$ représente un atome d'hydrogène, $R^8$ représente un atome d'hydrogène, et ses sels.

12. Le procédé selon la revendication 11, dans lequel on utilise le sel de triéthanolammonium.

13. Le procédé selon la revendication 10, dans lequel R représente le radical méthyle, $R^1$ représente la radical méthyle, $R^2$ représente un atome de chlore, $R^3$ représente un atome d'hydrogène, $R^4$ représente 4-$CH_3SO_2$—, $R^5$ représente un atome d'hydrogène, $R^6$ représente un atome d'hydrogène, $R^7$ représente un atome d'hydrogène, $R^8$ représente un atome d'hydrogène, et ses sels.